(19) Europäisches Patentamt
European Patent Office
Office européen des brevets

(11) Veröffentlichungsnummer: **0 291 799 B1**

(12) **EUROPÄISCHE PATENTSCHRIFT**

(45) Veröffentlichungstag der Patentschrift: **16.06.93**

(51) Int. Cl.$^5$: **C07D 405/04**, C07D 405/14, C07D 211/90, C07D 491/04, A61K 31/445

(21) Anmeldenummer: **88107382.9**

(22) Anmeldetag: **07.05.88**

Die Akte enthält technische Angaben, die nach dem Eingang der Anmeldung eingereicht wurden und die nicht in dieser Patentschrift enthalten sind.

# (54) Dioxyalkylenaryl-Dihydropyridine Zwischenprodukte zu ihrer Herstellung, Verfahren zu ihrer Herstellung und ihre Verwendung.

(30) Priorität: **19.05.87 DE 3716652**

(43) Veröffentlichungstag der Anmeldung:
**23.11.88 Patentblatt 88/47**

(45) Bekanntmachung des Hinweises auf die Patenterteilung:
**16.06.93 Patentblatt 93/24**

(84) Benannte Vertragsstaaten:
**AT BE CH DE ES FR GB IT LI NL SE**

(56) Entgegenhaltungen:
**EP-A- 0 197 488**
**DE-A- 2 819 788**
**DE-A- 2 916 358**
**DE-A- 3 248 548**
**US-A- 4 567 268**

**CHEMICAL ABSTRACTS, Band 105, Nr. 19, 10. November 1986, Columbus, Ohio, USA, Zusammenfassung-Nr. 172299g**

(73) Patentinhaber: **BAYER AG**

**W-5090 Leverkusen 1 Bayerwerk(DE)**

(72) Erfinder: **Franckowiak, Gerhard, Dr.**
**Henselweg 10**
**W-5600 Wuppertal 1(DE)**
Erfinder: **Marhold, Albrecht, Dr.**
**Carl-Duisberg Strasse 329**
**W-5090 Leverkusen 1(DE)**
Erfinder: **Bechem, Martin, Dr.**
**Obere Bergerheide 4**
**W- 5600 Wuppertal 1(DE)**
Erfinder: **Gross, Rainer, Prof. Dr.**
**Platzhofstrasse 23**
**W- 5600 Wuppertal 1(DE)**
Erfinder: **Kayser, Michael, Dr.**
**Walter-Flex Strasse 18**
**W- 5090 Leverkusen 1(DE)**
Erfinder: **Schramm, Matthias, Dr.**
**Paffrather Strasse 38**
**W- 5000 Köln 80(DE)**

**EP 0 291 799 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**CHEMICALABSTRACTS, Band 102, Nr. 1, 7. Januar 1985, columbus, Ohio, USA, Zusammenfassung-Nr. 6240z**

**CHEMICAL ABSTRACTS, Band 88, Nr. 21, 22. Mai 1978, Columbus, Ohio, USA, Zusammenfassung-Nr. 152593a**

**CHEMICAL ABSTRACTS, Band 65, Nr. 13, 19. Dezember 1966, Columbus, Ohio, USA, Zusammenfassung-Nr. 20092h**

**Pharmazie heute, 104(3), 1983, Seiten 139-146, Cas : 76.126530k**

**Journal of Organic Chemistry, vol. 37, Nr. 4, 25 February 1972, p. 673**

Erfinder: **Thomas, Günther, Dr.**
**Via Campogalla 21/14**
**I-20020 Arese (Mi)(IT)**

**Beschreibung**

Die Erfindung betrifft neue Dioxyalkylenaryl-Dihydropyridine, Zwischenprodukte zu ihrer Herstellung, Verfahren zu ihrer Herstellung und ihre Verwendung in Arzneimitteln, insbesondere in kreislaufbeeinflussenden Arzneimitteln.

Es ist bereits bekannt, daS 1,2-Dihydropyridin-Derivate pharmakologische Eigenschaften besitzen (vgl. z.B. EP-A-197 488). Weiterhin sind Tetrahydrofuro(3,4-b)pyridin-Derivate mit pharmakologischen Wirkungen bekannt und Verfahren zu ihrer Herstellung [vgl. z.B. US-A-4 567 268 Synthesis 1984, (7), 617-618 (Engl)]. Weiterhin sind Methylendioxyphenyl-dihydropyridine mit pharmakologischer Wirkung bekannt (vgl. ES 536, 537 = Chem. Abstract 105:172299g).

Die neuen Verbindungen unterscheiden sich von den bekannten Verbindungen dadurch, daß die Alkylendioxy-Gruppe, die an den Phenylring in der Stellung 4 des Dihydropyridins ankondensiert ist, fluoriert ist.

Die vorliegende Erfindung betrifft Dioxyalkylenaryl-Dihydropyridine der allgemeinen Formel (I)

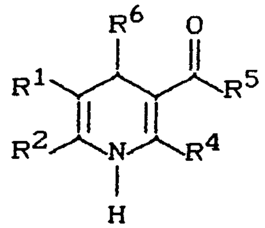

(I)

in welcher

$R^1$

- Wasserstoff, Cyano, Nitro oder
- für einen Rest der Formel $-CO_2R^7$ steht,
  wobei

$R^7$

- Wasserstoff oder geradkettiges, verzweigtes oder cyclisches Alkyl oder Alkenyl mit bis zu 16 Kohlenstoffatomen bedeutet, das gegebenenfalls durch ein Sauerstoffatom oder ein Schwefelatom in der Kette unterbrochen ist und/oder das gegebenenfalls substituiert ist durch ein oder mehrere, gleiche oder verschiedene Substituenten aus der Gruppe: Halogen, Cyano, Hydroxy, $C_2$-$C_7$-Acyloxy, Nitro oder durch eine gegebenenfalls durch Halogen, Cyano, Di-$C_1$-$C_5$-alkylamino, $C_1$-$C_6$-Alkyl, $C_1$-$C_6$-Alkoxy, Trifluormethyl oder Nitro substituierte Phenyl-, Phenoxy-, Phenylthio-oder Phenylsulfonylgruppe oder durch eine Heteroarylgruppe der Reihe Pyridyl, Thienyl, Furyl, Chinolyl oder Pyrimidyl, oder durch eine Aminogruppe, wobei diese Aminogruppe zwei gleiche oder verschiedene Substituenten aus der Gruppe $C_1$-$C_6$-Alkyl, $C_6$-$C_{12}$-Aryl, $C_7$-$C_{14}$-Aralkyl trägt oder wobei diese Substituenten gegebenenfalls mit dem Stickstoffatom einen 5- bis 7-gliedrigen Ring bilden, der als weiteres Heteroatom ein Schwefel- oder Sauerstoffatom oder die N-Phenyl- oder N-Alkylgruppierung enthalten kann, wobei die Alkylgruppe bis zu 4 Kohlenstoffatome umfassen kann

oder wobei

$R^7$

- eine direkte Bindung zu $R^2$ darstellt (für $R^2 \neq$ Cyano, Phenyl oder Formyl),

$R^2$, $R^4$

- gleich oder verschieden sind und jeweils
- für geradkettiges, verzweigtes oder cyclisches Alkyl mit bis zu 8 Kohlenstoffatomen stehen,
- für Phenyl oder Benzyl stehen, oder
- einer der Substituenten $R^2$ oder $R^4$ für geradkettiges oder verzweigtes Alkyl mit bis zu 6 Kohlenstoffatomen steht, das durch Halogen, $C_2$-$C_7$-Acyloxy, Hydroxy, Amino, Phthalimido, Alkoxy, Dialkoxy, Aminoalkoxy, Phthalimidoalkoxy, Piperidinoalkyl, Morpholinoalkoxy oder N-Phenyl-N-piperazinoalkoxy mit jeweils bis zu 6 Kohlenstoffatomen je Alkoxygruppe substituiert ist, oder für Formyl oder Cyano steht,

$R^5$

- für geradkettiges, verzweigtes oder cyclisches Alkyl mit bis zu 8 Kohlenstoffatomen steht, oder
- für eine Gruppe $-OR^8$ steht,
  wobei

$R^8$

- Wasserstoff oder geradkettiges, verzweigtes oder cyclisches Alkyl oder Alkenyl mit bis zu 16 Kohlenstoffatomen bedeutet, das gegebenenfalls durch ein Sauerstoffatom oder ein Schwefelatom in der Kette unterbrochen ist und/oder das gegebenenfalls substituiert ist durch ein oder mehrere gleiche oder verschiedene Substituenten aus der Gruppe Halogen, Cyano, Hydroxy, $C_2$-$C_7$-Acyloxy, Nitro oder durch eine Gruppe der Formel

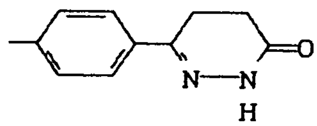

oder durch eine gegebenenfalls durch Halogen, Cyano, Di-$C_1$-$C_5$-alkylamio, $C_1$-$C_6$-Alkoxy, $C_1$-$C_6$-Alkoxy, $C_1$-$C_6$-Alkyl, Trifluormethyl oder Nitro substituierte Phenyl-, Phenoxy-, Phenylthio- oder Phenylsulfonylgruppe, oder durch eine Heteroarylgruppe der Reihe Pyridyl, Thienyl, Furyl, Chinolyl oder Pyrimidyl, oder durch eine Aminogruppe, wobei diese Aminogruppe zwei gleiche oder verschiedene Substituenten aus der Gruppe $C_1$-$C_6$-Alkyl, $C_6$-$C_{14}$-Aryl, $C_7$-$C_{14}$-Aralkyl trägt oder wobei diese Substituenten gegebenenfalls mit dem Stickstoffatom einen 5- bis 7-gliedrigen Ring bilden, der als weiteres Heteroatom ein Sauerstoff- oder Schwefelatom oder die N-Phenyl- oder N-Alkylgruppierung enthalten kann, wobei die Alkylgruppe bis zu 4 Kohlenstoffatome umfassen kann,

und

$R^6$

- für eine Gruppe der Formel

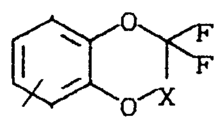

steht,
worin

X

- eine direkte Bindung oder $-CF_2$- oder -CHF-bedeutet,

sowie deren physiologisch unbedenkliche Salze.

Bevorzugte Verbindungen der allgemeinen Formel (I) sind solche, in denen

$R^1$

- für Wasserstoff, Nitro oder einen Rest der Formel $-CO_2R^7$ steht,
  wobei

$R^7$

- Wasserstoff, geradkettiges oder verzweigtes Alkyl mit bis zu 12 Kohlenstoffatomen bedeutet, das gegebenenfalls durch ein Sauerstoffatom in der Kette unterbrochen ist und/oder das gegebenenfalls ein- oder mehrfach substituiert ist durch Fluor, Chlor, Brom, Cyano, Hydroxy, Acetyloxy, oder durch eine gegebenenfalls durch Fluor, Chlor, Brom, Iod, Cyano, Nitro, $C_1$-$C_4$-Alkyl, $C_1$-$C_4$-Alkoxy oder Trifluormethyl substituierte Phenyl- oder Phenoxygruppe, oder durch eine $\alpha$-, $\beta$- oder $\gamma$-Pyridylgruppe, oder durch eine Aminogruppe, wobei diese Aminogruppe zwei gleiche oder verschiedene Substituenten aus der Reihe $C_1$-$C_4$-Alkyl, Phenyl oder Benzyl trägt,

oder

$R^7$

- eine direkte Bindung zu $R^2$ darstellt (für $R^2 \neq$ Phenyl),

$R^2$, $R^4$

- gleich oder verschieden sind und jeweils
- für geradkettiges oder verzweigtes Alkyl mit bis zu 6 Kohlenstoffatomen, für Phenyl oder Benzyl stehen, oder
- einer der Substituenten $R^2$ oder $R^4$ für geradkettiges oder verzweigtes Alkyl mit bis zu 4 Kohlenstoffatomen steht, das gegebenenfalls ein- oder mehrfach durch Fluor, Chlor, Brom, Acetyloxy, Benzoyloxy, Methoxy, Hydroxy, Amino, Phthalimido, Aminoalkoxy oder Phthalimidoalkoxy mit jeweils bis zu 4 Kohlenstoffatomen je Alkoxygruppe substituiert ist,

$R^5$

- für geradkettiges oder verzweigtes Alkyl mit bis zu 4 Kohlenstoffatomen steht, oder
- für die Gruppe $-OR^8$ steht,
  wobei

$R^8$

- Wasserstoff oder geradkettiges oder verzweigtes Alkyl mit bis zu 12 Kohlenstoffatomen bedeutet, das gegebenenfalls durch ein Sauerstoffatom in der Kette unterbrochen ist und/oder das gegebenenfalls substituiert ist durch ein oder mehrere Substituenten aus der Gruppe Fluor, Chlor, Brom, Cyano, Hydroxy, Acetyloxy oder durch die Gruppe der Formel

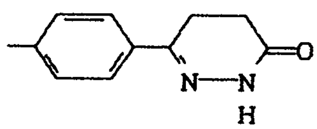

,

oder durch eine gegebenenfalls durch Fluor, Chlor, Brom, Iod, Cyano, Nitro, $C_1$-$C_4$-Alkyl, $C_1$-$C_4$-Alkoxy oder Trifluormethyl substituierte Phenyl- oder Phenoxygruppe oder durch eine $\alpha$-, $\beta$- oder $\gamma$-Pyridylgruppe, oder durch eine Aminogruppe, wobei diese Aminogruppe zwei gleiche oder verschiedene Substituenten aus der Reihe $C_1$-$C_4$-Alkyl, Phenyl oder Benzyl trägt,

und

$R^6$

- für eine Gruppe der Formel

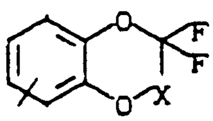

steht,
worin

X

- eine direkte Bindung oder $-CF_2-$ oder -CHF-bedeutet,

sowie deren physiologisch unbedenkliche Salze.

Besonders bevorzugt sind Verbindungen der allgemeinen Formel (I), in welcher

$R^1$

- für Nitro oder eine Gruppe der Formel $-CO_2R^7$ steht,
  worin

$R^7$

- Wasserstoff oder geradkettiges oder verzweigtes Alkyl mit bis zu 8 Kohlenstoffatomen bedeutet, das gegebenenfalls durch ein Sauerstoffatom in der Kette unterbrochen ist und/oder das gegebenenfalls substituiert ist durch bis zu 15 Fluoratomen, durch Chlor, Cyano, Acetyloxy, Phenyl, Phenoxy, $\alpha$-, $\beta$-, $\gamma$-Pyridyl oder durch eine Aminogruppe, wobei diese Aminogruppe zwei gleiche oder verschiedene Substituenten aus der Gruppe $C_1$-$C_4$-Alkyl oder Benzyl trägt,

oder

$R^7$

- eine direkte Bindung zu $R^2$ darstellt (für $R^2 \neq$ Phenyl),

$R^2$, $R^4$

- gleich oder verschieden sind und jeweils
- für geradkettiges oder verzweigtes Alkyl mit bis zu 4 Kohlenstoffatomen stehen, oder
- für Pydridyl stehen, oder
- einer der Substitueten $R^2$ oder $R^4$ für Alkyl mit bis zu 2 Kohlenstoffatomen steht, das gegebenenfalls durch bis zu 3 Fluor, durch Chlor, Brom, Acetoxy, Benzoyloxy, Hydroxy oder Aminoethoxy substituiert ist,

$R^3$

- für Wasserstoff steht,

$R^5$

- für die Gruppe $-OR^8$ steht, worin

$R^8$

- Wasserstoff oder geradkettiges oder verzweigtes Alkyl mit bis zu 8 Kohlenstoffatomen bedeutet, das gegebenenfalls durch ein Sauerstoffatom in der Kette unterbrochen ist und/oder das gegebenenfalls substituiert ist durch bis zu 15 Fluoratome durch Chlor, Cyano, Acetyloxy, oder durch die Gruppe der Formel

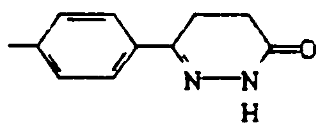

oder durch Phenyl, Phenoxy, α, β-oder γ-Pyridyl oder durch eine Aminogruppe, wobei diese Aminogruppe zwei gleiche oder verschiedene Substituenten aus der Gruppe $C_1$-$C_4$-Alkyl oder Benzyl trägt,

und

$R^6$

- für eine Gruppe der Formel

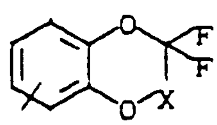

steht,
worin

X

- für eine direkte Bindung steht, oder
- für die Gruppen $-CF_2-$ und $-CHF-$ steht,

sowie deren physiologisch unbedenkliche Salze.

Physiologisch unbedenkliche Salze können Salze der erfindungsgemäßen Verbindungen mit anorganischen oder organischen Säuren sein. Als Beispiele seien genannt: Hydrohalogenide wie z.B. Hydrochloride, Hydrobromide, Hydrogensulfate, Sulfate, Hydrogenphosphate, Phosphate, oder Acetate, Maleate, Fumarate, Citrate, Tartrate, Lactate oder Benzoate.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Die erfindungsgemäßen Verbindungen sind neu und besitzen wertvolle pharmakologische Eigenschaften. Sie beeinflussen den Blutdruck und können somit zur Bekämpfung von Kreislauferkrankungen eingesetzt werden.

Die erfindungsgemäßen Verbindungen der allgemeinen Formel (I),
in welcher
$R^1$ - $R^6$
- die angegebene Bedeutung haben,
erhält man, indem man
[A] Aldehyde der allgemeinen Formel (II),

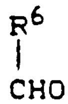

(II)

in welcher
$R^6$ die angegebene Bedeutung hat,
und Ketone der allgemeinen Formel (III)

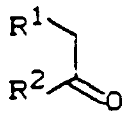

(III),

in welcher
$R^1$ und $R^2$ die angegebene Bedeutung haben,
oder deren Knoevenagel-Kondensationsprodukte (Ylidenverbindungen) der allgemeinen Formel (IV),

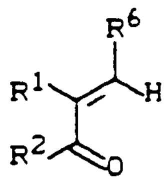

(IV)

in welcher
$R^1$, $R^2$, $R^6$ die oben angegebene Bedeutung haben,
mit Enaminen der allgemeinen Formel (V),

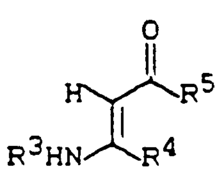

(V),

in welcher $R^3$ - $R^5$ die angegebene Bedeutung haben,
in inerten Lösemitteln umsetzt,
oder indem man
[B] Aldehyde der allgemeinen Formel (II) und Ketone der allgemeinen Formel (VI),

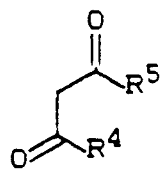

(VI),

in welcher
$R^4$ und $R^5$ die angegebene Bedeutung haben,
oder deren Knoevenagel-Kondensationsprodukte (Ylidenverbindungen) der allgemeinen Formel (VII)

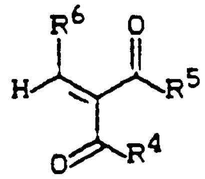

(VII),

in welcher
$R^4$, $R^5$, $R^6$ die oben angegebene Bedeutung haben,
mit Enaminen der allgemeinen Formel (VIII)

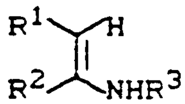

(VIII)

in welcher
$R^1$, $R^2$, $R^3$ die oben angegebene Bedeutung haben,
in inerten Lösemitteln umsetzt
und im Fall, daß $R^2$ eine direkte Bindung zu $R^7$ darstellt, die erhaltenen Dihydropyridine zu Lactonen cyclisiert.

Für den Fall, daß in der allgemeinen Formel I die Reste $R^1$, $R^3$, $R^5$, $R^6$, $R^7$, $R^8$ und X die oben angegebene Bedeutung haben und $R^2$ oder $R^4$ für die Formyl- oder die Nitrilgruppe oder für einen Alkylrest steht, der durch Hydroxy, Amino oder Aminoalkoxy substituiert ist, erfolgt die Herstellung der erfindungsgemäßen Verbindungen vorzugsweise derart, daß man geeignete erfindungsgemäße Zwischenprodukte synthetisiert und diese in Folgereaktionen weiter umsetzt. Steht $R^2$ oder $R^4$ beispielsweise für eine Formylgruppe, so erhält man diese Derivate durch saure Hydrolyse erfindungsgemäßer Verbindungen der allgemeinen Formel I, in der $R^2$ oder $R^4$ einen Dialkoxymethylrest bedeutet. Die erfindungsgemäßen Derivate der allgemeinen Formel I mit $R^2$ und/oder $R^4$ mit der Bedeutung Nitril werden erhalten durch Umsetzung der Aldehyde mit Hydroxylamin und anschließender Dehydratisierung des entstandenen Oxims nach literaturbekannten Methoden.

Auf ähnliche Weise erhält man die erfindungsgemäßen Verbindungen der allgemeinen Formel I, in welcher $R^2$ oder $R^4$ für einen Alkylrest steht, der durch a) Hydroxy, b) Amino oder c) Aminoalkoxy substituiert ist, indem man a) die entsprechenden erfindungsgemäßen Acyloxyderivate sauer oder alkalisch hydrolysiert, b) die entsprechenden nach oben beschriebenen Verfahren herstellbaren Phthalimido-Verbindungen beispielsweise hydrazinolysiert oder c) die entsprechenden erfindungsgemäßen Phthalimidoalkoxyderivate mit Hydrazin umsetzt.

Je nach Art der verwendeten Ausgangsstoffe können die Synthese-Varianten für die erfindungsgemäßen Verbindungen durch folgende Formelschemata wiedergegeben werden:

[A]

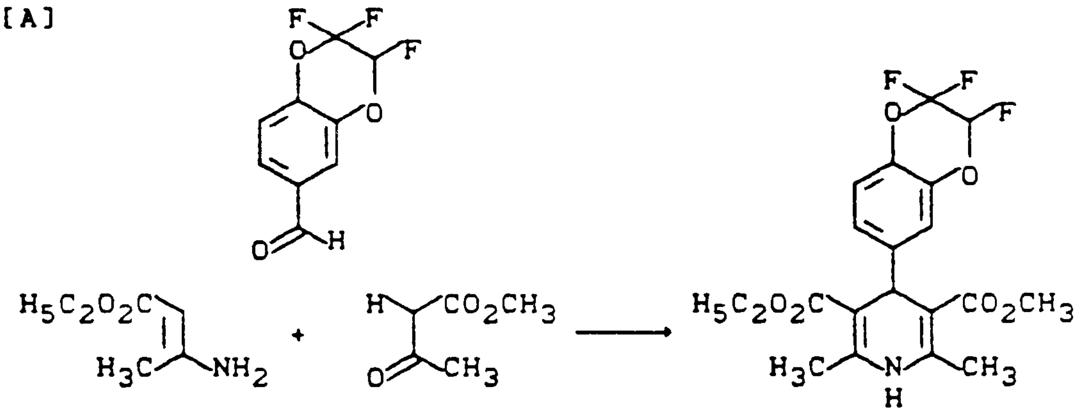

bzw.

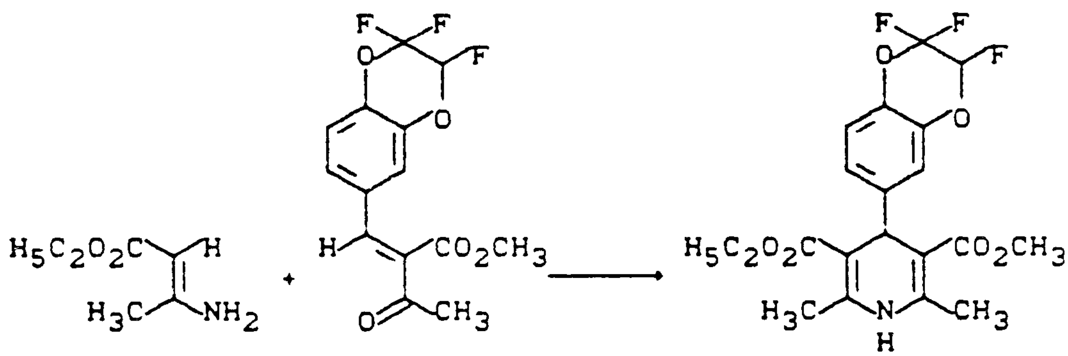

[B]

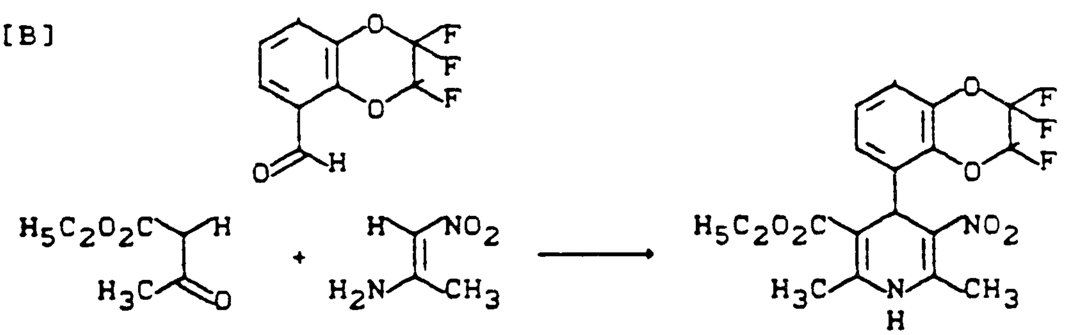

bzw.

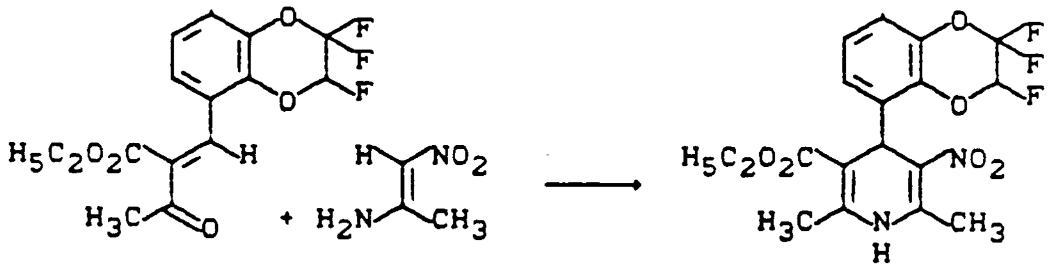

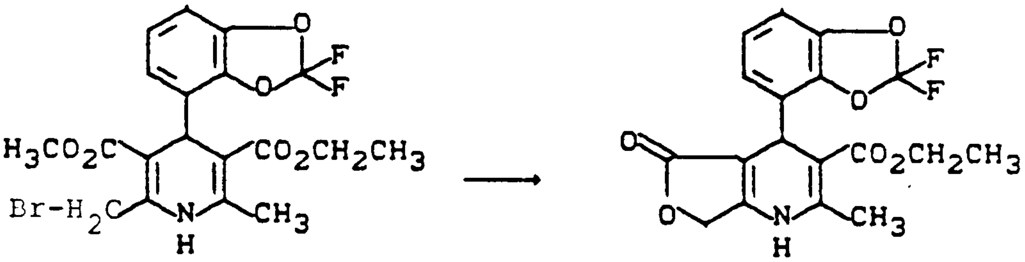

Verfahrensvarianten A und B

Als Lösemittel für die erfindungsgemäßen Verfahrensvarianten A, B kommen alle inerten organischen Lösemittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether, oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen +20 °C und +150 °C, vorzugsweise zwischen +20 °C und +100 °C, insbesondere bei der Siedetemperatur des jeweiligen Lösemittels.

Die Umsetzung kann bei Normaldruck, aber auch bei erhöhtem oder erniedrigtem Druck durchgeführt werden. Im allgemeinen arbeitet man unter Normaldruck.

Bei der Durchführung der erfindungsgemäßen Verfahren A und B werden die an der Reaktion beteiligten Stoffe bevorzugt jeweils in molaren Mengen eingesetzt.

Die Cyclisierung der Dihydropyridine zu Lactonen kann durchgeführt werden, indem man solche Dihydropyridine in welchen $R^3$ für einen Acyloxymethylrest steht in inerten Lösemitteln, gegebenenfalls in Anwesenheit von Basen cyclisiert, oder indem man solche Dihydropyridine in welchen $R^3$ für einen Halogenmethylrest steht mit oder ohne Lösemittel pyrolysiert.

Als Basen für die Cyclisierung eignen sich die üblichen Basen wie zum Beispiel Alkali- oder Erdalkalihydroxide, besonders Natrium-, Kalium-, Calciumhydroxid, oder Amine wie Ammoniak, Triethylamin, Pyridin. Die Cyclisierung kann in den üblichen Lösemitteln wie aromatische Kohlenwasserstoffe (z.B. Benzol, Toluol), Alkoholen (z.B. Ethanol, Propanol, Methanol) oder Essigsäure durchgeführt werden. Die Cyclisierung erfolgt bei Temperaturen von +10 °C bis +200 °C, bevorzugt von +20 °C bis +150 °C.

Die Pyrolyse kann mit oder ohne Lösemittel durchgeführt werden. Als Lösemittel kommen gegebenenfalls alle üblichen inerten organischen Lösemittel in Frage. Dazu zählen bevorzugt Kohlenswasserstoffe wie Benzol, Toluol oder Xylol, Tetralin, Erdölfraktionen, Ether wie Diethylether, Dioxan, Tetrahydrofuran, Glykolmono- bzw. Diethylether, Halogenkohlenwasserstoffe wie Di-, Tri-, oder Tetrachlormethan, Dichlor- oder Trichlorethylen.

Die Pyrolyse wird in einem Temperaturbereich von +20 °C bis +300 °C, bevorzugt von +40 °C bis +250 °C durchgeführt.

Die Pyrolyse kann bei normalem, erhöhtem oder erniedrigten Druck durchgeführt werden. Im allgemeinen arbeitet man bei Normaldruck. Bevorzugt ist das Verfahren F.

Die Aldehyde der allgemeinen Formel (II)

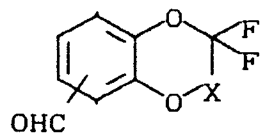

(II)

in welcher

X

- für eine direkte Bindung, oder
- für eine Gruppe der Formel $-CF_2-$ oder -CHF- steht,

sind, ausgenommen die Verbindungen 2,2-Difluor-5-formyl-1,3-benzodioxol (vgl. J. Org. Chemie 1972, 37 (4), 673 Engl.) und 3,4-Trifluorethylendioxy-benzaldehyd (vgl. DE-A-2 916 358), neu und können hergestellt werden, indem man

[C] Methylsubstituierte Verbindungen der allgemeinen Formel (IX)

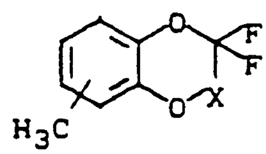

(IX)

in welcher

X die oben angegebene Bedeutung hat,

in inerten Lösemitteln mit Halogenierungsmitteln, gegebenenfalls in Anwesenheit von Radikalbildnern, umsetzt,

und dann die Halogenmethylverbindungen der allgemeinen Formel (X)

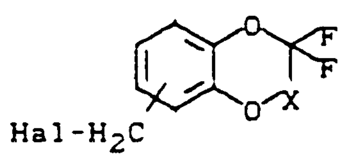

(X)

in welcher

X die oben angegebene Bedeutung hat

und

Hal für Halogen, bevorzugt für Chlor oder Brom steht,

in inerten Lösemitteln, gegebenenfalls in Anwesenheit von Säuren, mit Urotropin behandelt,

oder indem man

[D] Aminoverbindungen der allgemeinen Formel (XI)

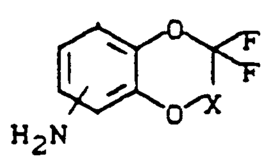

(XI)

in welcher

X die oben angegebene Bedeutung hat,

in inerten Lösemitteln, in Anwesenheit von Säuren mit Nitriten umsetzt,

und dann die erhaltenen Diazoniumsalze mit Formaldoxim umsetzt.

Die erfindungsgemäßen Verfahren können durch folgendes Schema verdeutlicht werden:

[C] 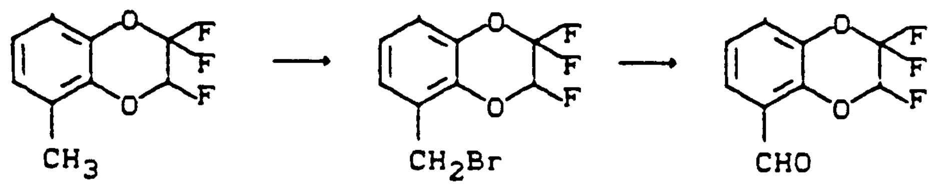

[D] 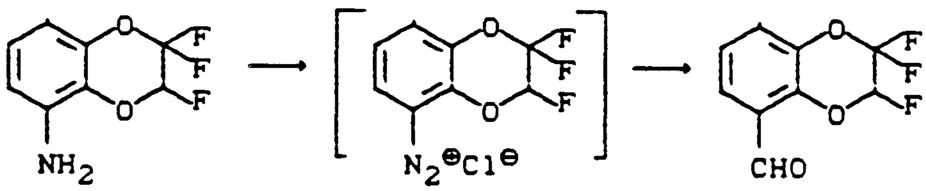

Verfahrensvariante C

Als Lösemittel eignen sich hierfür alle inerten organischen Lösemittel, die sich unter den Reaktionsbedingungen nicht verändern. Hierzu gehören bevorzugt Halogenkohlenwasserstoffe wie beispielsweise Dichlormethan, Trichlormethan oder Tetrachlorkohlenstoff, oder Kohlenwasserstoffe wie Benzol, Toluol oder Xylol, oder Eisessig.

Als Halogenierungsmittel können die allgemein üblichen Halogenierungsmittel verwendet werden. Bevorzugt sind Chlor, Brom, N-Chlorsuccinimid (NCS) oder N-Bromsuccinimid (NBS), gegebenenfalls in Anwesenheit von Radikalbildnern wie Azobisisobuttersäurenitril (AIBN), Benzoylperoxid, Porofor-N oder Licht. Besonders bevorzugt ist die Bromierung mit NBS oder Porofor-N in Tetrachlormethan.

Die Reaktionstemperaturen können im allgemeinen in einem größeren Bereich variiert werden. Bevorzugt arbeitet man in einem Bereich von -10 °C bis +100 °C, bevorzugt von 0° bis +80 °C.

Die Umsetzung kann bei Normaldruck, aber auch bei erhöhtem oder bei erniedrigtem Druck durchgeführt werden. Im allgemeinen arbeitet man bei normalem Druck.

Das Mengenverhältnis der Reaktanden ist im allgemeinen beliebig. Bevorzugt arbeitet man jedoch mit einer Menge von 1 bis 5 Mol, besonders bevorzugt von 1 mol des Halogenierungsmittels bezogen auf 1 mol der methylsubstituierten Verbindung.

Die Umsetzung der Brommethylverbindung mit Urotropin im zweiten Schritt der erfindungsgemäßen Verfahrensvariante C erfolgt im allgemeinen in Wasser oder organischen Lösemitteln wie Alkoholen, bevorzugt Methanol, Ethanol, Propanol oder Isopropanol, oder deren Gemischen, in Anwesenheit einer Säure, bevorzugt einer anorganischen Mineralsäure wie beispielsweise Salzsäure, Bromwasserstoffsäure oder Schwefelsäure. Bevorzugt führt man das Verfahren in Wasser in Anwesenheit von Salzsäure durch.

Die Umsetzung erfolgt im allgemeinen in einem Temperaturbereich von 0 °C bis +150 °C, bevorzugt von +20 °C bis +100 °C.

Die Umsetzung kann bei Normaldruck, aber auch bei erhöhtem oder erniedrigtem Druck durchgeführt werden. Im allgemeinen arbeitet man bei Normaldruck.

Die Aufarbeitung des Reaktionsgemisches erfolgt nach üblichen Methoden durch Destillation (bevorzugt Wasserdampfdestillation), Kristallisation und/oder Chromatographie.

Die als Ausgangsstoffe eingesetzten methylsubstituierten Verbindungen der allgemeinen Formel (IX) sind bekannt oder können nach bekannten Methoden hergestellt werden.

Verfahrensvariante D

Bei der Durchführung der erfindungsgemäßen Verfahrensvariante D entstehen im allgemeinen die Diazoniumsalze als Zwischenprodukte. Es hat sich hierbei als zweckmäßig erwiesen, das Verfahren ohne Isolierung der Zwischenprodukte durchzuführen.

Als Lösemittel eignet sich hierbei Wasser, oder Alkohole wie Methanol, Ethanol, Propanol oder Isopropanol, oder Amide wie Dimethylformamid oder Dimethylacetamid, oder Säuren wie Mineralsäuren

oder Carbonsäuren. Bevorzugt sind Wasser und/oder Säuren wie Salzsäure, Bromwasserstoffsäure, Schwefelsäure, Phosphorsäure oder Essigsäure. Ebenso ist es möglich, Gemische der genannten Lösemitteln einzusetzen.

Als Säuren werden im allgemeinen Mineralsäuren eingesetzt. Bevorzugt werden hierbei Chlorwasserstoffsäure, Bromwasserstoffsäure, Schwefelsäure, Phosphorsäure oder aber Gemische der genannten Säuren.

Besonders bevorzugt wird die Diazotierung in einem Gemisch aus Wasser und konzentrierter Salzsäure durchgeführt.

Als Nitrite werden im allgemeinen Alkylnitrite wie beispielsweise Natrium- oder Kaliumnitrit verwendet. Bevorzugt wird Natriumnitrit eingesetzt.

Die erfindungsgemäße Verfahrensvariante D wird im allgemeinen derart durchgeführt, daß man zunächst eine Lösung der Diazoniumsalze herstellt, die in einem zweiten Schritt mit Formaldoxim umgesetzt werden.

Die Umsetzung wird im allgemeinen in einem Temperaturbereich von -10°C bis +100°C, bevorzugt von 0°C bis +80°C durchgeführt.

Die Umsetzung wird im allgemeinen bei Normaldruck durchgeführt. Es ist ebenso möglich bei erhöhtem oder erniedrigtem Druck zu arbeiten.

Die als Ausgangsstoffe eingesetzten Amine der allgemeinen Formel (XI) sind bekannt oder können nach bekannten Methoden hergestellt werden [Europäische Patentschrift 11 179].

Die als Ausgangsstoffe eingesetzten Ketone der allgemeinen Formel III bzw. VI sind bekannt oder können nach bekannten Methoden hergestellt werden [vgl. z.B. D. Borrmann, "Umsetzung von Diketen mit Alkoholen, Phenolen und Mercaptanen", in Houben-Weyls "Methoden der Organischen Chemie",Vol. VII/4, 230 ff (1968); Y. Oikawa, K. Sugano und O. Yonemitsu, J. Org. Chem. 43, 2087 (1978); N. Levy, C.W. Scaife, J. Chem. Soc. (London) 1946, 1100; C.D. Hurd, M.W. Nilson, J. Org. Chem. 20, 927 (1955); S. Gelin, P. Pollet Synth. Commun. 1980, 805 bzw. Tetrahedron 34, 1453 (1978); G.F. Field, W.J. Zally, Synthesis 1979, 295].

Die als Ausgangsstoffe eingesetzten Enamine der allgemeinen Formel V bzw. VIII sind bekannt oder können nach bekannten Methoden hergestellt werden [vgl. z.B. A.C. Cope, J. Am. Soc. 67, 1017 (1945); H. Böhme, K.-H. Weisel, Arch. Pharm 310, 30 (1977)].

Die vorstehenden Herstellungsverfahren sind lediglich zur Verdeutlichung angegeben. Die Herstellung der Verbindungen der Formel I ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen beeinflussen die Kontraktionskraft des Herzens und den Tonus der glatten Muskulatur. Sie können deshalb in Arzneimitteln zur Beeinflussung des pathologisch veränderten Blutdrucks, als Koronartherapeutika und zur Behandlung der Herzinsuffizienz, eingesetzt werden. Darüberhinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden.

Dazu werden die Herzen von 250 bis 350 g schweren Meerschweinchen verwendet. Der Thorax wird geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. Als Perfusionsmedium dient eine Krebs-Henseleit-Lösung (1) (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l $Na_2EDTA$), deren $CaCl_2$-Gehalt 1,2 mmol/l beträgt. Als energielieferndes Substrat werden 10 mmol/l Glucose zugesetzt. vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32°C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert (Opie, L., J. Physiol. 180 (1965), 529 - 541). Der Perfusionsdruck wird mittels eines Druckaufnehmers, der vor dem Herzen mit dem Perfusionssystem in Verbindung steht, registriert. Unter diesen Bedingungen zeigt eine Senkung des Perfusionsdrucks eine Koronardilatation, eine Zu- bzw. Abnahme der linksventrikulären Kontraktionsamplitude einen Anstieg bzw. eine Senkung der Herzkontraktilität an. Die erfindungsgemäßen Verbindungen werden in geeigneten Verdünnungen in das Perfusionsmedium kurz vor dem isolierten Herzen infundiert.

Die folgenden Werte zeigen beispielhaft den Effekt der erfindungsgemäßen Verbindungen am isoliert perfundierten Meerschweinchenherzen, ausgedrückt als prozentuale Differenz gegenüber dem gleich 100% gesetzten Ausgangswert.

| Bsp.-Nr. | Konzentration (g/l) | %-Änderung der Ventrikelamplitude |
|---|---|---|
| 8 | $10^{-3}$ | - 23% |
| 10 | $10^{-3}$ | -100% |
| 14 | $10^{-3}$ | -100% |
| 15 | $10^{-3}$ | - 79% |
| 19 | $10^{-3}$ | - 20% |
| 20 | $10^{-3}$ | - 4% |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B.: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze

überschritten werden muß. Im Falle der Applikation größerer Mengen kann es empfehlenswert sein, diese in mehreren Einzelgaben über den Tag zu verteilen.

Herstellungsbeispiele

Beispiel 1

1,4-Dihydro-2,6-dimethyl-4-(2,2,3-trifluor-1,4-benzodioxan-6-yl)-pyridin-3,5-dicarbonsäuredimethylester

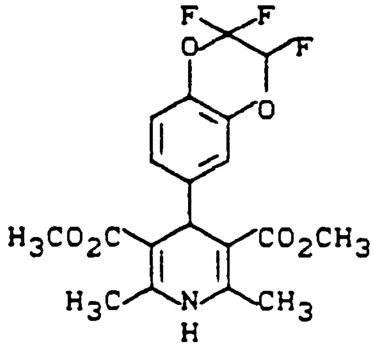

10 mmol Acetessigsäuremethylester, 10 mmol β-Aminocrotonsäuremethylester und 10 mmol 2,2,3-Trifluor-1,4-benzodioxan-6-yl-carbaldehyd werden 12 h in 30 ml Methanol zum Rückfluß erhitzt. Nach Prüfen auf vollständige Umsetzung wird das Methanol teilweise abgedampft. Unter Zusatz von Petrolether und Anreiben kristallisiert das Produkt langsam aus.
Ausbeute: 59% der Theorie
Schmp.: 227 °C

Beispiel 2

(2,2-Difluor-1,3-benzodioxol-5-yl)-methylen-acetessigsäure-methylester

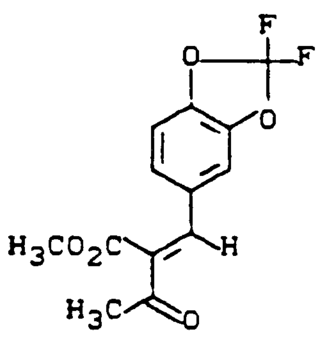

50 mmol 5-(Butyliminomethyl)-2,2-difluor-1,3-benzdioxol und 50 mmol Acetessigsäuremethylester werden gleichzeitig in 80 ml Acetanhydrid gegeben und nach kurzem Erwärmen 24 h bei Raumtemperatur stehen gelassen. Der Ansatz wird dann mit 1 l Eiswasser hydrolysiert, das ausgefallene Öl wird abgetrennt, mit etwas Methylenchlorid verdünnt, getrocknet und als Rohprodukt weiter eingesetzt.

Beispiel 3

4-(2,2-Difluor-1,3-benzodioxol-5-yl)-1,4-dihydro-2,6-dimethyl-pyridin-3,5-dicarbonsäure-dimethylester

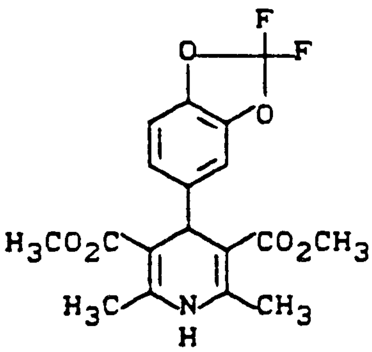

20 mmol β-Aminocrotonsäuremethylester und 20 mmol roher (2,2-Difluor-1,3-benzodioxol-5-yl)-methylen-acetessigsäuremethylester werden in 30 ml Methanol 12 h zum Rückfluß erhitzt, das Methanol wird abgedampft und der Rückstand an Kieselgel mit Chloroform chromatographiert. Die eingedampfte Produktfraktion wird in wenig Methylenchlorid aufgenommen und durch schnelles Evakuieren zu einem Glas aufgeschäumt.
Schmp.: Harz
Ausbeute: 69% der Theorie

Beispiel 4

2-[(2,2,3-Trifluor-1,4-benzodioxan-5-yl)-methylen]-4-acetoxy-acetessigsäureethylester

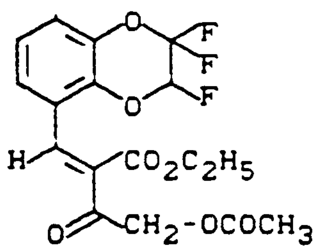

50 mmol 5-(Butyliminomethyl-2,2,3-trifluor-1,4-benzodioxan und 50 mmol 4-Acetoxy-acetessigsäureethylester werden in 80 ml Acetanhydrid 24 h gerührt. Der Ansatz wird mit ca. 1 l Eiswasser hydrolysiert, das ausgefallene Öl wird abgetrennt, mit Methylenchlorid aufgenommen, getrocknet und als Rohprodukt eingesetzt.

Beispiel 5

2-Acetoxymethyl-1,4-dihydro-6-methyl-4-(2,2,3-trifluor-1,4-benzodioxan-5-yl)-pyridin-3,5-dicarbonsäurediethylester

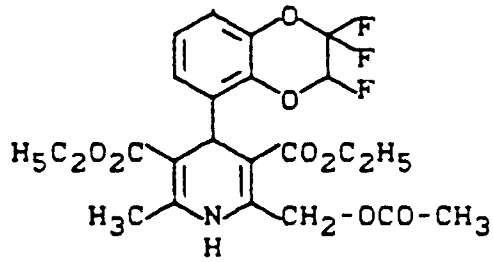

30 mmol β-Aminocrotonsäureethylester und 35 mmol roher 2-[2,2,3-Trifluor-1,4-benzodioxan-5-yl)-methylen]-4-acetoxy-acetessigsäureethylester werden in 50 ml Ethanol 6 h zum Rückfluß erhitzt. Das Lösemittel wird abgedampft, der Rückstand an Kieselgel mit Chloroform / Methanol chromatographiert. Die eingedampften Produktfraktionen werden mit Methylenchlorid aufgenommen und durch schnelles Evakuieren zu einem Glas aufgeschäumt.
Schmp.: Harz
Ausbeute: 52% der Theorie

Beispiel 6

2-Methyl-5-oxo-4-(2,2,3-trifluor-1,4-benzodioxan-5-yl)-1,4,5,7-tetrahydrofuro[3,4-b]pyridin-3-carbonsäureethylester

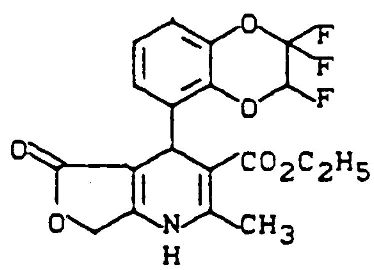

10 mmol 2-Acetoxymethyl-1,4-dihydro-6-methyl-4-(2,2,3-trifluor-1,4-benzodioxan-5-yl)-pyridin-3,5-dicarbonsäurediethylester werden in 25 mmol ethanolischer Salzsäure 0,5 Stunden erhitzt. Das Lösemittel wird abgedampft, der Rückstand an wenig Ethanol unter Zusatz von Petrolether kristallisiert.
Schmp.: 142 ° C
Ausbeute: 79% der Theorie

Beispiel 7

4-(2,2-Difluor-1,3-benzodioxol-5-yl)-1,4-dihydro-6-methyl-5-nitro-pyridin-3-carbonsäuremethylester

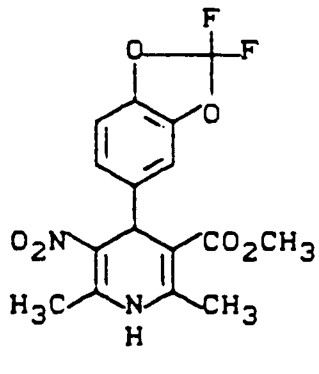

20 mmol (2,2-Difluor-1,3-benzodioxol-5-yl)-methylenacetessigsäuremethylester aus Beispiel 2 werden in 30 ml Methanol unter Zusatz von 2 ml gesättigter methanolischer Ammoniaklösung zum Rückfluß erhitzt und portionsweise mit 40 mmol Nitroaceton versetzt. Es wird noch 6 h zum Rückfluß erhitzt, das Lösemittel abgedampft und der Rückstand auf Kieselgel mit Chloroform / Methanol chromatographiert. Die Produktfraktionen werden nach dem Eindampfen im Methylenchlorid aufgenommen und durch schnelles Evakuieren in einen glasartigen Schaum überführt.

Schmp.: Harz

Ausbeute: 48% der Theorie

Analog Beispiel 1 bis 7 wurden die weiteren Beispiele der Tabelle 1 erhalten. Die Beispiele der Tabelle 2 werden analog Beispiel 1, 2 oder 5 erhalten.

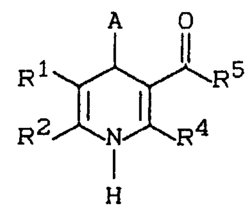

| Bsp.-Nr. | $R^1$ | $R^2$ | $R^4$ | $R^5$ | A | Schmp. [°C] |
|---|---|---|---|---|---|---|
| 8 | $-COOCH_3$ | $-CH_3$ | $-CH_3$ | $-OCH_3$ | 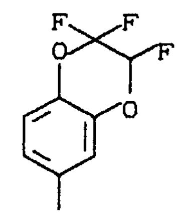 | 227 |
| 9 | $-COOCH_3$ | $-CH_3$ | $-CH_3$ | $-OCH_3$ | 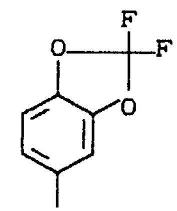 | 185 |
| 10 | $-COOCH_3$ | $-CH_3$ | $-CH_3$ | $-OCH_3$ | 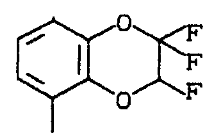 | 219 |

| Bsp.-Nr. | $R^1$ | $R^2$ | $R^4$ | $R^5$ | A | Schmp. [°C] |
|---|---|---|---|---|---|---|
| 11 | $-COOCH_3$ | $-CH_3$ | $-CH_3$ | $-OCH_3$ | 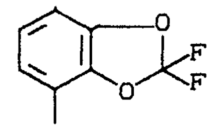 | Harz |
| 12 | $-COOCH(CH_3)_2$ | $-CH_3$ | $-CH_3$ | $-OCH_3$ | 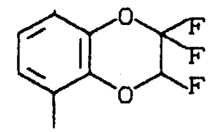 | 133 |
| 13 | $-COOC_{10}H_{21}$ | $-CH_3$ | $-CH_3$ | $-OCH_3$ | 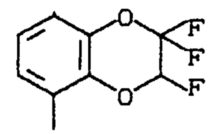 | Harz |
| 14 | $-COO-(CH_2)_3-C_6F_{13}$ | $-CH_3$ | $-CH_3$ | $-OCH_3$ | 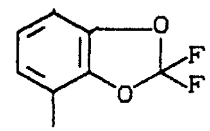 | Harz |
| 15 | $-COO-(CH_2)_3-C_6F_{13}$ | $-CH_3$ | $-CH_3$ | $-OC_2H_5$ | 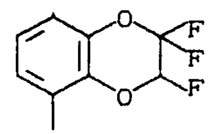 | Harz |

| Bsp.-Nr. | $R^1$ | $R^2$ | $R^4$ | $R^5$ | A | Schmp. [°C] |
|---|---|---|---|---|---|---|
| 16 | -COO———— | -$CH_2$ | -$CH_3$ | -$OC_2H_5$ | 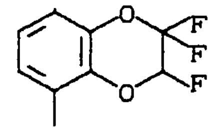 | 142 |
| 17 | -$NO_2$ | -$CH_3$ | -$CH_3$ | -$OCH_3$ | 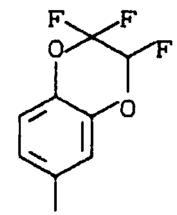 | 189 |
| 18 | -$NO_2$ | -$CH_3$ | -$CH_3$ | -$OCH_3$ | 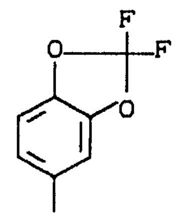 | Harz |
| 19 | -$NO_2$ | -$CH_3$ | -$CH_3$ | -$OCH_3$ | 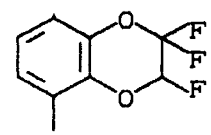 | 223 |

| Bsp.-Nr. | $R^1$ | $R^2$ | $R^4$ | $R^5$ | A | Schmp. [°C] |
|---|---|---|---|---|---|---|
| 20 | $-NO_2$ | $-CH_3$ | $-CH_3$ | $-OCH_3$ | 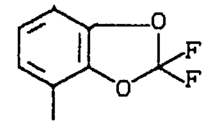 | 173 |
| 21 | $-NO_2$ | $-CH_3$ | $-CH_3$ | $-O-CH(CH_3)_2$ | 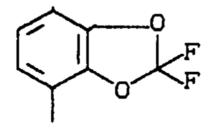 | 156 |
| 22 | $-NO_2$ | $-CH_3$ | $-CH_3$ | $-CH_2CH_2CN$ | 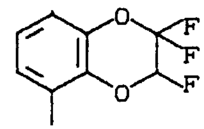 | Harz |
| 23 | $-NO_2$ | $-CH_3$ | $-CH_3$ | 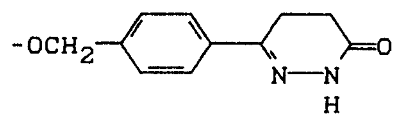 | 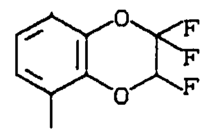 | 228 |
| 24 | $-NO_2$ | $-CH_3$ | $-CH_3$ | 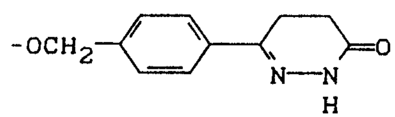 | 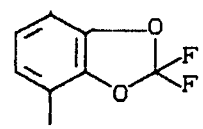 | 268 |

Beispiel 25

Synthese von 2,2-Difluor-4-formyl-1,3-benzodioxol

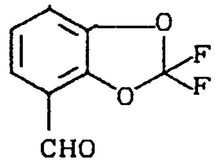

a) 4-Methyl-1,3-benzodioxol

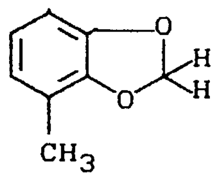

In einer Rührapparatur werden 400 g Dibrommethan und 50 g Tetrabutylammoniumbromid bei Rückfluß vorgelegt und eine Lösung aus 400 ml Wasser, 130 g Natriumhydroxid und 200 g 3-Methylbenzkatechin innerhalb von 5 h zugetropft. Nach Ende der Zugabe wird noch für 2 h nachgerührt, abgekühlt und mit Dichlormethan extrahiert. Die organische Phase wird getrocknet und an einer Kolonne destilliert. Nach einem Vorlauf von nicht umgesetzten Dibrommethan werden 175 g 4-Methyl-1,3-benzodioxol erhalten.
Kp.: 94 °C (85 mbar)
$[\alpha]^D$ = 1,5218
b) 2,2-Dichlor-4-methyl-1,3-benzodioxol

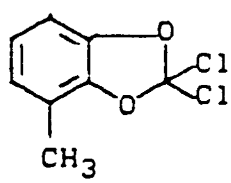

Zu 276 g Phosphorpentachlorid in 840 ml Tetrachlormethan (hergestellt aus Phosphortrichlorid und Chlor) werden unter Rühren 160 g 4-Methyl-1,3-benzodioxol getropft. Es setzt sofort zügig Chlorwasserstoff-Entwicklung ein. Gegen Ende der Reaktion werden bei ca. 20 °C 85 g Chlor eingeleitet und anschließend bei Ende der Gasentwicklung auf Rückfluß erhitzt. Durch Destillation werden das Phosphortrichlorid und der Tetrachlorkohlenstoff zurückgewonnen und anschließend das 4-Methyl-2,2-dichlor-1,3-benzodioxol bei Kp.: 98 - 99 °C (22 mbar) erhalten.
Ausbeute: 235 g
c) 2,2-Difluor-4-Methyl-1,3-benzodioxol

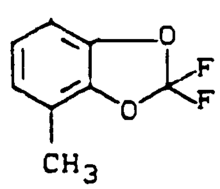

Man legt in einer Fluorierungsapparatur 200 ml wasserfreien Fluorwasserstoff vor und tropft bei ca. 0°C 235 g Chlorverbindung aus 2,2-Dichlor-4-methyl-1,3-benzodioxol zu. Nach Ende der Zugabe erwärmt man auf 18 - 20°C und rührt bis zum Ende der Chlorwasserstoff-Entwicklung nach. Dann wird der überschüssige Fluorwasserstoff überdestilliert und der Rückstand mit Natriumfluorid verrührt und abgesaugt. Das Rohprodukt wird über eine Drehbandkolonne abdestilliert und man erhält 142 g 2,2-Difluor-4-methyl-1,3-benzodioxol.

Kp.: 52 - 54°C (bei 16 mbar)

$n_D^{20}$ = 1.4500

d) 4-Brommethyl-2,2-difluor-1,3-benzodioxol

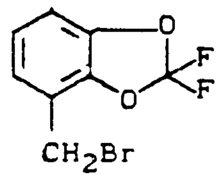

Zu einer Suspension von 90 g N-Bromsuccinimid in 400 ml Tetrachlormethan gibt man 76 g 2,2-Difluor-4-methyl-1,3-benzodioxol und 200 mg Azobisisobutyronitril. Dann wird unter Rühren auf Rückfluß erhitzt bis das gesamte ungelöste Material oben schwimmt. Nach Abkühlen wird abgesaugt, das Succinimid mit Tetrachlormethan nachgewaschen und anschließend die Lösung destilliert, um das Tetrachlormethan abzutrennen. Es verbleiben im Rückstand 117 g rohes Benzylbromid, das ohne Reinigung eingesetzt wird.

e) 2,2-Difluor-4-formyl-1,3-benzodioxol

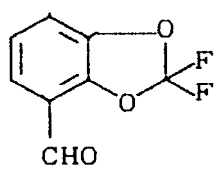

Zu 120 g Urotropin in 250 ml Wasser werden 117 g rohes 4-Brommethyl-2,2-difluor-1,3-benzodioxol gegeben und für 3 Stunden auf Rückfluß erhitzt. Danach werden 120 ml Salzsäure in 150 ml Wasser zugegeben und für 3 weitere Stunden erhitzt. Anschließend wird das Rohprodukt mit Wasserdampf übergetrieben, aus der Vorlage abgetrennt und mit 100 g Natriumbisulfat in 250 ml Wasser geschüttelt. Nach Extraktion mit Dichlormethan wird die wäßrige Phase mit 2 n Schwefelsäure sauer gestellt, der Aldehyd abgetrennt und destilliert. Die Ausbeute beträgt 53 g.

Kp.: 99 - 101°C (bei 18 mbar)

$n_D^{20}$ = 1,4875

Beispiel 26

Synthese von 2,2,3-Trifluor-6-formyl-1,4-benzodioxan

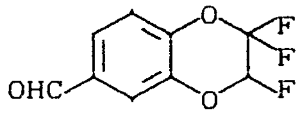

a) 2,2,3-Trifluor-6-methyl-1,4-benzodioxan

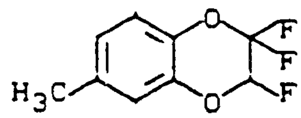

In 1500 ml Tetramethylensulfon werden 600 g 4-Methylbrenzkatechin vorgelegt und 500 g Kaliumhydroxid portionsweise eingetragen. Man gibt 20 ml Wasser hinzu und rührt bei 110 °C bis nahezu alles gelöst ist. Dann wird Difluorchlorethan im Maße der Aufnahme bei starkem Rühren bis zur Sättigung eingeleitet. Dann wird Wasserdampf eingeleitet und das Produkt übergetrieben. Nach Abtrennen und Trocknung der organischen Phase wird diese destilliert. Es werden 820 g Produkt erhalten .
Kp.: 76 - 80 °C (bei 16 mbar),
$n_D^{20}$ = 1,4590 (Das Produkt enthält als Nebenprodukt 2,3,3,-Trifluor-6-methyl-1,4-benzodioxan).
b) 6-Brommethyl-2,2,3-trifluor-1,4-benzodioxan / 6-Dibrommethyl-2,2,3-trifluor-1,4-benzodioxan

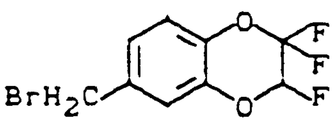
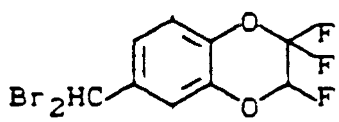

430 g 2,2,3-Trifluor-6-methyl-1,4-benzodioxan werden wie in Beispiel 1d) in 3 l Tetrachlormethan mit 750 g N-Bromsuccinimid und 5 g Azobisisobutyronitril bromiert. Man erhält 710 g einer Mischung aus Mono- und Dibromid.
Kp.: 115 - 118 °C (bei 10 mbar)
c) 6-Formyl-2,2,3-trifluor-1,4-benzodioxan

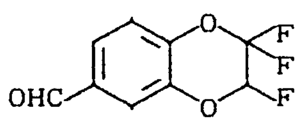

Zu 318 g Urotropin in 630 ml Wasser werden 318 g 6-Brommethyl-2,2,3-trifluor-1,4-benzodioxan gegeben und für 2 h auf Rückfluß erhitzt, anschließend wird die Reaktionsmischung mit 318 ml Salzsäure und 470 ml Wasser versetzt und für weitere 3 Stunden erhitzt. Durch Wasserdampf wird das Produkt übergetrieben, abgetrennt und nach Trocknung destilliert. Man erhält 158 g Destillat (Kp.: 112 - 115 °C (bei 18 mbar)), das beim Stehen teilweise kristallisiert. Man saugt den Kristallbrei ab und wäscht die Kristalle mit kaltem Petrolether.
Ausbeute: 108 g 2,2,3-Trifluor-6-formyl-1,4-benzodioxan.
Schmp.: 92 °C
(Der Aldehyd ist spektroskopisch rein).
Aus dem Filtrat läßt sich durch Destillation an einer Drehbandkolonne auch das 2,2,3-Trifluor-6-formyl-1,4-benzodioxan isolieren.
Kp.: 119 - 120 °C (bei 20 mbar)

Beispiel 27

Synthese von 2,2,3,3-Tetrafluor-5-formyl-1,4-benzodioxan

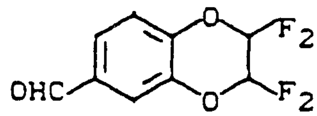

Man legt in 800 ml Wasser und 250 ml konz. Salzsäure 222 g 5-Amino-2,2,3,3-tetrafluor-1,4-benzodioxan vor, erwärmt auf 80 °C, kühlt unter Rühren schnell auf 0 °C ab und diazoliert dann durch Zutropfen von 69 g Natriumnitrat in 150 ml Wasser. Dann wird eine Stunde bei 10 °C und 20 Minuten bei 20 °C nachgerührt. In einer zweiten Rührapparatur werden 130 g Hydroxylaminhydrochlorid und 60 g Paraformaldehyd in 400 ml Wasser erwärmt, bis eine klare Lösung entsteht. Anschließend werden 235 g Natriumacetat, 40 g Kupfersulfat und 13 g Natriumsulfit zugegeben, 800 g Eis eingetragen und anschließend mit 500 g Natriumacetat gepuffert. Zu dieser Lösung wird bei 5 - 10 °C die zuerst hergestellte Diazoniumsalz-Lösung getropft. Nach Ende der Zugabe wird noch 1 h bei 20 °C gerührt und dann langsam auf 100 °C erhitzt und das Produkt mit Wasserdampf übergetrieben. Nach Abtrennen des Rohprodukts aus dem Destillat wird getrocknet und über eine Drehbandkolonne redestilliert. Man erhält 108 g Aldehyd.
Kp.: 85 - 87 °C (bei 14 mbar)
$n_D^{20}$ = 1,4685

Beispiel 28

5-(Butyliminomethyl)-2,2-difluor-1,3-benzodioxol

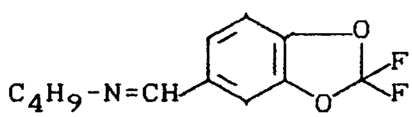

0,10 Mol 2,2-Difluor-5-formyl-1,3-benzodioxol werden in 150 ml Methylenchlorid gelöst und unter leichter Kühlung bei Raumtemperatur mit 0,12 Mol n-Butylamin versetzt. Man läßt 20 h ohne Rühren bei Raumtemperatur stehen, destilliert das Lösemittel samt dem abgeschiedenen Wasser bei leichtem Vakuum ab, versetzt erneut mit 150 ml Methylenchlorid, destilliert dieses ab und trocknet das zurückbleibende Öl im Ölpumpenvakuum. Die Butyliminoverbindung kann roh, z.B. in Beispiel 2 eingesetzt werden.

Butyliminoverbindungen weiterer erfindungsgemäßer Aldehyde werden analog Beispiel 26 hergestellt.

**Patentansprüche**

**1.** Dioxyalkylenaryl-Dihydropyridine der allgemeinen Formel (I)

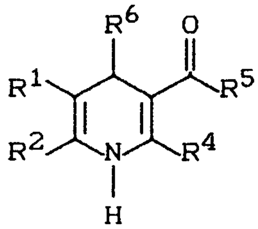

(I)

in welcher
$R^1$

- Wasserstoff, Cyano, Nitro oder
- für einen Rest der Formel $-CO_2R^7$ steht, wobei

$R^7$

- Wasserstoff oder geradkettiges, verzweigtes oder cyclisches Alkyl oder Alkenyl mit bis zu 16 Kohlenstoffatomen bedeutet, das gegebenenfalls durch ein Sauerstoffatom oder ein Schwefelatom in der Kette unterbrochen ist und/oder das gegebenenfalls substituiert ist durch ein oder mehrere gleiche oder verschiedene Substituenten aus der Gruppe Halogen, Cyano, Hydroxy, $C_2$-$C_7$-Acyloxy, Nitro oder durch eine gegebenenfalls durch Halogen, Cyano, Di-$C_1$-$C_5$-alkylamino, $C_1$-$C_6$-Alkyl, $C_1$-$C_6$-Alkoxy, Trifluormethyl oder Nitro substituierte Phenyl-, Phenoxy-, Phenylthio- oder Phenylsulfonylgruppe oder durch eine Heteroarylgruppe der Reihe Pyridyl, Thienyl, Furyl, Chinolyl oder Pyrimidyl, oder durch eine Aminogruppe, wobei diese Aminogruppe zwei gleiche oder verschiedene Substituenten aus der Gruppe $C_1$-$C_6$-Alkyl, $C_6$-$C_{12}$-Aryl, $C_7$-$C_{14}$-Aralkyl trägt oder wobei diese Substituenten gegebenenfalls mit dem Stickstoffatom einen 5- bis 7-gliedrigen Ring bilden, der als weiteres Heteroatom ein Schwefel- oder Sauerstoffatom oder die N-Phenyl- oder N-Alkylgruppierung enthalten kann, wobei die Alkylgruppe bis zu 4 Kohlenstoffatome umfassen kann

oder wobei

$R^7$

- eine direkte Bindung zu $R^2$ darstellt (für $R^2 \neq$ Cyano, Phenyl oder Formyl),

$R^2$, $R^4$

- gleich oder verschieden sind und jeweils
- für geradkettiges, verzweigtes oder cyclisches Alkyl mit bis zu 8 Kohlenstoffatomen stehen,
- für Phenyl oder Benzyl stehen, oder
- einer der Substituenten $R^2$ oder $R^4$ für geradkettiges oder verzweigtes Alkyl mit bis zu 6 Kohlenstoffatomen steht, das durch Halogen, $C_2$-$C_7$-Acyloxy, Hydroxy, Amino, Phthalimido, Alkoxy, Dialkoxy, Aminoalkoxy, Phthalimidoalkoxy, Piperidinoalkyl, Morpholinoalkoxy oder N-Phenyl-N-piperazinoalkoxy mit jeweils bis zu 6 Kohlenstoffatomen je Alkoxygruppe substituiert ist, oder für Formyl oder Cyano steht,

$R^5$

- für geradkettiges, verzweigtes oder cyclisches Alkyl mit bis zu 8 Kohlenstoffatomen steht,
  oder
- für eine Gruppe $-OR^8$ steht, wobei

$R^8$

- Wasserstoff oder geradkettiges, verzweigtes oder cyclisches Alkyl oder Alkenyl mit bis zu 16 Kohlenstoffatomen bedeutet, das gegebenenfalls durch ein Sauerstoffatom oder ein Schwefelatom in der Kette unterbrochen ist und/oder das gegebenenfalls substituiert ist durch ein oder mehrere gleiche oder verschiedene Substituenten aus der Gruppe Halogen, Cyano, Hydroxy, $C_2$-$C_7$-Acyloxy, Nitro oder durch eine Gruppe der Formel

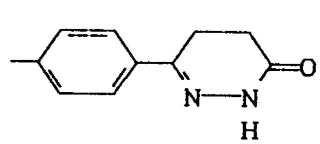

oder durch eine gegebenenfalls durch Halogen, Cyano, Di-$C_1$-$C_5$-alkylamio, $C_1$-$C_6$-Alkoxy, $C_1$-$C_6$-Alkoxy, $C_1$-$C_6$-Alkyl, Trifluormethyl oder Nitro substituierte Phenyl-, Phenoxy-, Phenylthio- oder Phenylsulfonylgruppe, oder durch eine Heteroarylgruppe der Reihe Pyridyl, Thienyl, Furyl, Chinolyl oder Pyrimidyl, oder durch eine Aminogruppe, wobei diese Aminogruppe zwei gleiche oder verschiedene Substituenten aus der Gruppe $C_1$-$C_6$-Alkyl, $C_6$-$C_{14}$-Aryl, $C_7$-$C_{14}$-Aralkyl, oder wobei diese Substituenten gegebenenfalls mit dem Stickstoffatom einen 5- bis 7-gliedrigen Ring

bilden, der als weiteres Heteroatom ein Sauerstoff- oder Schwefelatom oder die N-Phenyl-oder N-Alkylgruppierung enthalten kann, wobei die Alkylgruppe bis zu 4 Kohlenstoffatome umfassen kann,

und

$R^6$

- für eine Gruppe der Formel

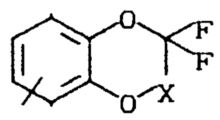

steht,

worin

X

- eine direkte Bindung oder $-CF_2-$ oder -CHF-bedeutet,

sowie deren physiologisch unbedenkliche Salze.

**2.** Verbindungen der allgemeinen Formel (I) gemäß Anspruch 1, in welcher

$R^1$

- für Wasserstoff, Nitro oder einen Rest der Formel $-CO_2R^7$ steht, wobei

$R^7$

- Wasserstoff, geradkettiges oder verzweigtes Alkyl mit bis zu 12 Kohlenstoffatomen bedeutet, das gegebenenfalls durch ein Sauerstoffatom in der Kette unterbrochen ist und/oder das gegebenenfalls ein- oder mehrfach substituiert ist durch Fluor, Chlor, Brom, Cyano, Hydroxy, Acetyloxy, oder durch eine gegebenenfalls durch Fluor, Chlor, Brom, Iod, Cyano, Nitro, $C_1$-$C_4$-Alkyl, $C_1$-$C_4$-Alkoxy oder Trifluormethyl substituierte Phenyl- oder Phenoxygruppe, oder durch eine $\alpha$-, $\beta$- oder $\gamma$-Pyridylgruppe, oder durch eine Aminogruppe, wobei diese Aminogruppe zwei gleiche oder verschiedene Substituenten aus der Reihe $C_1$-$C_4$-Alkyl, Phenyl oder Benzyl trägt,

oder

$R^7$

- eine direkte Bindung zu $R^2$ darstellt (für $R^2 \neq$ Phenyl),

$R^2$, $R^4$

- gleich oder verschieden sind und jeweils
- für geradkettiges oder verzweigtes Alkyl mit bis zu 6 Kohlenstoffatomen, für Phenyl oder Benzyl stehen, oder
- einer der Substituenten $R^2$ oder $R^4$ für geradkettiges oder verzweigtes Alkyl mit bis zu 4 Kohlenstoffatomen steht, das gegebenenfalls ein- oder mehrfach durch Fluor, Chlor, Brom, Acetyloxy, Benzoyloxy, Methoxy, Hydroxy, Amino, Phthalimido, Aminoalkoxy oder Phthalimidoalkoxy mit jeweils bis zu 4 Kohlenstoffatomen je Alkoxygruppe substituiert ist,

$R^5$

- für geradkettiges oder verzweigtes Alkyl mit bis zu 4 Kohlenstoffatomen steht, oder
- für die Gruppe $-OR^8$ steht, wobei

$R^8$

- Wasserstoff oder geradkettiges oder verzweigtes Alkyl mit bis zu 12 Kohlenstoffatomen bedeutet, das gegebenenfalls durch ein Sauerstoffatom in der Kette unterbrochen ist und/oder das gegebenenfalls substituiert ist durch ein oder mehrere Substituenten aus der Gruppe Fluor, Chlor, Brom, Cyano, Hydroxy, Acetyloxy oder durch die Gruppe der Formel

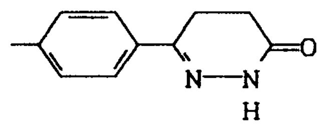 ,

oder durch eine gegebenenfalls durch Fluor, Chlor, Brom, Iod, Cyano, Nitro, $C_1$-$C_4$-Alkyl, $C_1$-$C_4$-Alkoxy oder Trifluormethyl substituierte Phenyl- oder Phenoxygruppe oder durch eine α-, β- oder γ-Pyridylgruppe, oder durch eine Aminogruppe, wobei diese Aminogruppe zwei gleiche oder verschiedene Substituenten aus der Reihe $C_1$-$C_4$-Alkyl, Phenyl oder Benzyl trägt,

und

$R^6$
- für eine Gruppe der Formel

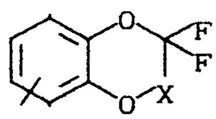

steht,
worin

X
- eine direkte Bindung oder $-CF_2-$ oder -CHF-bedeutet,

sowie deren physiologisch unbedenkliche Salze.

**3.** Verbindungen der allgemeinen Formel (I) gemäß Anspruch 1, in welcher

$R^1$
- für Nitro oder eine Gruppe der Formel $-CO_2R^7$ steht, worin

$R^7$
- Wasserstoff oder geradkettiges oder verzweigtes Alkyl mit bis zu 8 Kohlenstoffatomen bedeutet, das gegebenenfalls durch ein Sauerstoffatom in der Kette unterbrochen ist und/oder das gegebenenfalls substituiert ist durch bis zu 15 Fluoratomen, durch Chlor, Cyano, Acetyloxy, Phenyl, Phenoxy, α-, β-, γ-Pyridyl oder durch eine Aminogruppe, wobei diese Aminogruppe zwei gleiche oder verschiedene Substituenten aus der Gruppe $C_1$-$C_4$-Alkyl oder Benzyl trägt,

oder

$R^7$
- eine direkte Bindung zu $R^2$ darstellt (für $R^2 \neq$ Phenyl),

$R^2$, $R^4$
- gleich oder verschieden sind und jeweils
- für geradkettiges oder verzweigtes Alkyl mit bis zu 4 Kohlenstoffatomen stehen, oder
- einer der Substituenten $R^2$ oder $R^4$ für Alkyl mit bis zu 2 Kohlenstoffatomen steht, das gegebenenfalls durch bis zu 3 Fluor, durch Chlor, Brom, Acetoxy, Benzoyloxy, Hydroxy oder Aminoethoxy substituiert ist,

$R^5$
- für die Gruppe $-OR^8$ steht, worin

$R^8$
- Wasserstoff oder geradkettiges oder verzweigtes Alkyl mit bis zu 8 Kohlenstoffatomen bedeutet, das gegebenenfalls durch ein Sauerstoffatom in der Kette unterbrochen ist und/oder das gegebenenfalls substituiert ist durch bis zu 15 Fluoratome durch Chlor, Cyano, Acetyloxy, oder durch die Gruppe der Formel

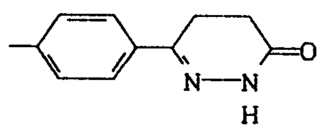

oder durch Phenyl, Phenoxy, α-, β- oder γ-Pyridyl oder durch eine Aminogruppe, wobei diese Aminogruppe zwei gleiche oder verschiedene Substituenten aus der Gruppe $C_1$-$C_4$-Alkyl oder Benzyl trägt,

und

$R^6$

- für die eine Gruppe der Formel

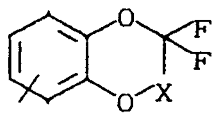

steht,
worin

X

- für eine direkte Bindung steht, oder
- für die Gruppen $-CF_2-$ und $-CHF-$ steht,

sowie deren physiologisch unbedenkliche Salze.

**4.** Verfahren zur Herstellung von Verbindungen der allgemeinen Formel (I)

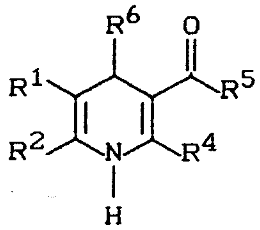

(I)

in welcher

$R^1$

- Wasserstoff, Cyano, Nitro oder
- für einen Rest der Formel $-CO_2R^7$ steht,
  wobei

$R^7$

- Wasserstoff oder geradkettiges, verzweigtes oder cyclisches Alkyl oder Alkenyl mit bis zu 16 Kohlenstoffatomen bedeutet, das gegebenenfalls durch ein Sauerstoffatom oder ein Schwefelatom in der Kette unterbrochen ist und/oder das gegebenenfalls substituiert ist durch ein oder mehrere gleiche oder verschiedene Substituenten aus der Gruppe Halogen, Cyano, Hydroxy, $C_2$-$C_7$-Acyloxy, Nitro oder durch eine gegebenenfalls durch Halogen, Cyano, Di-$C_1$-$C_5$-alkylamino, $C_1$-$C_6$-Alkyl, $C_1$-$C_6$-Alkoxy, Trifluormethyl oder Nitro substituierte Phenyl-, Phenoxy-, Phenylthio- oder Phenylsulfonylgruppe oder durch eine Heteroarylgruppe der Reihe Pyridyl, Thienyl, Furyl, Chinolyl oder Pyrimidyl, oder durch eine Aminogruppe, wobei diese Aminogruppe zwei gleiche oder verschiedene Substituenten aus der Gruppe $C_1$-$C_6$-Alkyl, $C_6$-$C_{12}$-Aryl, $C_7$-$C_{14}$-Aralkyl trägt oder wobei diese Substituenten gegebenenfalls mit dem Stickstoffatom einen 5- bis 7-gliedrigen Ring bilden, der als weiteres Heteroatom ein Schwefel- oder Sauerstoffatom oder die N-Phenyl- oder N-Alkylgruppierung enthalten kann, wobei die Alkylgruppe bis zu 4 Kohlenstoffatome umfassen kann

oder wobei

$R^7$

- eine direkte Bindung zu $R^2$ darstellt (für $R^2 \neq$ Cyano, Phenyl oder Formyl),

$R^2$, $R^4$

- gleich oder verschieden sind und jeweils
- für geradkettiges, verzweigtes oder cyclisches Alkyl mit bis zu 8 Kohlenstoffatomen stehen,
- für Phenyl oder Benzyl stehen, oder
- einer der Substituenten $R^2$ oder $R^4$ für geradkettiges oder verzweigtes Alkyl mit bis zu 6 Kohlenstoffatomen steht, das durch Halogen, $C_2$-$C_7$-Acyloxy, Hydroxy, Amino, Phthalimido, Alkoxy, Dialkoxy, Aminoalkoxy, Phthalimidoalkoxy, Piperidinoalkyl, Morpholinoalkoxy oder N-Phenyl-N-piperazinoalkoxy mit jeweils bis zu 6 Kohlenstoffatomen je Alkoxygruppe substituiert ist, oder für Formyl oder Cyano steht,

$R^5$

- für geradkettiges, verzweigtes oder cyclisches Alkyl mit bis zu 8 Kohlenstoffatomen steht, oder
- für eine Gruppe $-OR^8$ steht,
  wobei

$R^8$

- Wasserstoff oder geradkettiges, verzweigtes oder cyclisches Alkyl oder Alkenyl mit bis zu 16 Kohlenstoffatomen bedeutet, das gegebenenfalls durch ein Sauerstoffatom oder ein Schwefelatom in der Kette unterbrochen ist und/oder das gegebenenfalls substituiert ist durch ein oder mehrere gleiche oder verschiedene Substituenten aus der Gruppe Halogen, Cyano, Hydroxy, $C_2$-$C_7$-Acyloxy, Nitro oder durch eine Gruppe der Formel

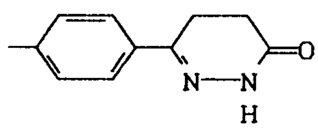

oder durch eine gegebenenfalls durch Halogen, Cyano, Di-$C_1$-$C_5$-alkylamio, $C_1$-$C_6$-Alkoxy, $C_1$-$C_6$-Alkoxy, $C_1$-$C_6$-Alkyl, Trifluormethyl oder Nitro substituierte Phenyl-, Phenoxy-, Phenylthio- oder Phenylsulfonylgruppe, oder durch eine Heteroarylgruppe der Reihe Pyridyl, Thienyl, Furyl, Chinolyl oder Pyrimidyl, oder durch eine Aminogruppe, wobei diese Aminogruppe zwei gleiche oder verschiedene Substituenten aus der Gruppe $C_1$-$C_6$-Alkyl, $C_6$-$C_{14}$-Aryl, $C_7$-$C_{14}$-Aralkyl, oder wobei diese Substituenten gegebenenfalls mit dem Stickstoffatom einen 5- bis 7-gliedrigen Ring bilden, der als weiteres Heteroatom ein Sauerstoff- oder Schwefelatom oder die N-Phenyl-oder N-Alkylgruppierung enthalten kann, wobei die Alkylgruppe bis zu 4 Kohlenstoffatome umfassen kann,

und

$R^6$

- für eine Gruppe der Formel

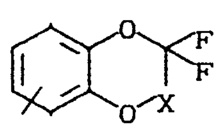

steht,
worin

X

- eine direkte Bindung oder $-CF_2-$ oder -CHF-bedeutet,

dadurch gekennzeichnet, daß man

[A] Aldehyde der allgemeinen Formel (II),

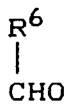

(II)

in welcher
$R^6$ die angegebene Bedeutung hat,
und Ketone der allgemeinen Formel (III)

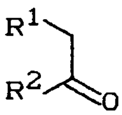

(III),

in welcher
$R^1$ und $R^2$ die angegebene Bedeutung haben,
oder deren Knoevenagel-Kondensationsprodukte (Ylidenverbindungen) der allgemeinen Formel (IV),

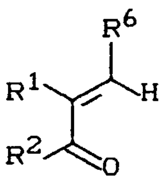

(IV)

in welcher
$R^1$, $R^2$, $R^6$ die oben angegebene Bedeutung haben,
mit Enaminen der allgemeinen Formel (V),

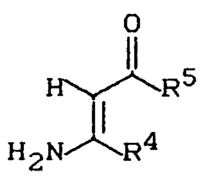

(V),

in welcher $R^4$ und $R^5$ die angegebene Bedeutung haben,
in inerten Lösemitteln umsetzt,
oder indem man
[B] Aldehyde der allgemeinen Formel (II) und Ketone der allgemeinen Formel (VI),

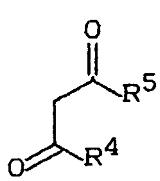

(VI),

in welcher
$R^4$ und $R^5$ die angegebene Bedeutung haben,
oder deren Knoevenagel-Kondensationsprodukte (Ylidenverbindungen) der allgemeinen Formel (VII)

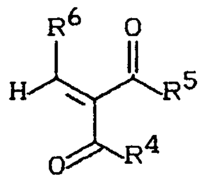

(VII),

in welcher
$R^4$, $R^5$, $R^6$ die oben angegebene Bedeutung haben,
mit Enaminen der allgemeinen Formel (VIII)

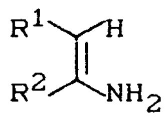

(VIII)

in welcher
$R^1$ und $R^2$ die oben angegebene Bedeutung haben,
in inerten Lösemitteln bei Temperaturen zwischen 20° und 150°C umsetzt,
wobei für den Fall, daß $R^2$ eine direkte Bindung zu $R^7$ bedeutet, die erhaltenen Dihydropyridine zu Lactonen cyclisiert werden.

**5.** Verfahren zur Herstellung von Dihydropyridin-Lactonen der Formel I, in denen $R^2$ eine direkte Bindung zu $R^7$ bedeutet, dadurch gekennzeichnet, daß man Dihydropyridine der allgemeinen Formel (I),
in welcher
$R^1$ für $CO_2R^7$ steht,
$R^2$ für einen Acyloxymethylrest steht,
in inerten organischen Lösungsmitteln, gegebenenfalls in Anwesenheit von Basen cyclisiert
oder
in welcher
$R^2$ für einen Halogenmethylrest steht,
gegebenenfalls in Anwesenheit von Lösemitteln bei Temperaturen zwischen 20 und 300°C pyrolysiert, wobei die übrigen Substituenten die im Anspruch 4 angegebene Bedeutung haben.

**6.** Verbindungen der allgemeinen Formel (I) gemäß Anspruch 1 zur Verwendung bei der Bekämpfung von Kreislauferkrankungen.

**7.** Arzneimittel enthaltend mindestens eine Verbindung der allgemeinen Formel (I) gemäß Anspruch 1.

**8.** Verfahren zur Herstellung von Arzneimitteln, dadurch gekennzeichnet, daß man Verbindungen der allgemeinen Formel (I) gemäß Anspruch 1, gegebenenfalls unter Verwendung von üblichen Hilfs- und Trägerstoffen in eine geeignete Applikationsform überführt.

**9.** Verwendung von Verbindungen der allgemeinen Formel (I) gemäß Anspruch 1 bei der Herstellung von Arzneimitteln gegen Kreislauferkrankungen.

**10.** Aldehyde der allgemeinen Formel (II)

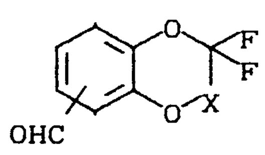

(II)

in welcher

X

- für eine direkte Bindung, oder
- für eine Gruppe der Formel $-CF_2-$ oder -CHF-steht,

ausgenommen die Verbindungen 2,2-Difluor-5-formyl-1,3-benzodioxol und 3,4-Trifluorethylendioxybenzaldehyd.

**11.** 2,2-Difluor-4-formyl-1,3-benzodioxol der Formel

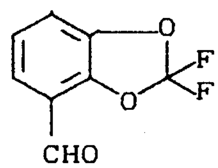

**12.** 2,2,3,3-Tetrafluor-5-formyl-1,4-benzo-dioxan der Formel

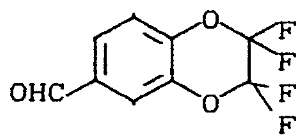

**13.** Verfahren zur Herstellung von Aldehyden der allgemeinen Formel (II)

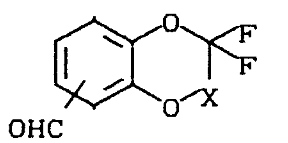

(II)

in welcher

X für eine direkte Bindung, oder
für eine Gruppe der Formel $-CF_2-$ oder -CHF-steht,

ausgenommen die Verbindungen 2,2-Difluor-5-formyl-1,3-benzodioxol und 3,4-Trifluorethylendioxy-benzaldehyd,

[a] Methylsubstituierte Verbindungen der allgemeinen Formel (IX)

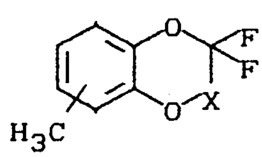

(IX)

in welcher

X die oben angegebene Bedeutung hat,

in inerten Lösemitteln mit Halogenierungsmitteln, gegebenenfalls in Anwesenheit von Radikalbildnern, umsetzt,

und dann die Halogenmethylverbindungen der allgemeinen Formel (X)

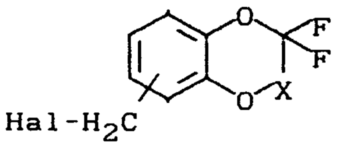

(X)

in welcher
X die oben angegebene Bedeutung hat
und
Hal für Halogen steht,
in inerten Lösemitteln, gegebenenfalls in Anwesenheit von Säuren, mit Urotropin behandelt,
oder indem man
[b] Aminoverbindungen der allgemeinen Formel (XI)

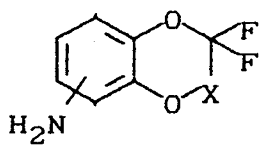

(XI)

in welcher
X die oben angegebene Bedeutung hat,
in inerten Lösemitteln, in Anwesenheit von Säuren mit Nitriten umsetzt,
und dann die erhaltenen Diazoniumsalze mit Formaldoxim umsetzt.

## Claims

1. Dioxyalkylenearyl-dihydropyridines of the general formula (I)

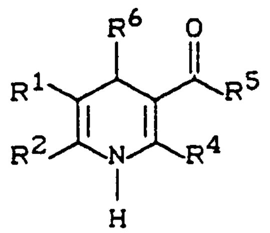

(I)

in which

$R^1$ represents hydrogen, cyano or nitro, or represents a radical of the formula $-CO_2R^7$, wherein

$R^7$ denotes hydrogen or straight-chain, branched or cyclic alkyl or alkenyl which has up to 16 carbon atoms and is optionally interrupted in the chain by an oxygen atom or a sulphur atom and/or is optionally substituted by one or more identical or different substituents from the group comprising halogen, cyano, hydroxyl, $C_2$-$C_7$-acyloxy and nitro or by a phenyl, phenoxy, phenylthio or phenylsulphonyl group which is optionally substituted by halogen, cyano, di-$C_1$-$C_5$-alkylamino, $C_1$-$C_6$-alkyl, $C_1$-$C_6$-alkoxy, trifluoromethyl or nitro, or by a heteroaryl group of the series comprising pyridyl, thienyl, furyl, quinolyl or pyrimidyl, or by an amino group, this amino group carrying two identical or different substituents from the group comprising $C_1$-$C_6$-alkyl, $C_6$-$C_{12}$-aryl and $C_7$-$C_{14}$-aralkyl, or it being possible for these substituents, together with the nitrogen atom, optionally to form a 5- to 7- membered ring which can contain, as a further hetero atom, a sulphur or oxygen atom or the N-phenyl or N-alkyl grouping, it being possible for the alxyl group to contain up to 4 carbon atoms,

or wherein

$R^7$ represents a direct bond to $R^2$ (where $R^2 \neq$ cyano, phenyl or formyl),

$R^2$, $R^4$ are identical or different and in each case represent straight-chain, branched or cyclic alkyl with up to 8 carbon atoms, or represent phenyl or benzyl, or one of the substituents $R^2$ or $R^4$ represents straight-chain or branched alkyl which has up to 6 carbon atoms and is substituted by halogen, $C_2$-$C_7$-acryloxy, hydroxyl, amino, phthalimido, alkoxy, dialkoxy, aminoalkoxy, phthalimidoalkoxy, piperidinoalkyl, morpholinoalkoxy or N-phenyl-N-piperazinoalkoxy with in each case up to 6 carbon atoms per alkoxy group, or represents formyl or cyano,

$R^5$ represents straight-chain, branched or cyclic alkyl with up to 8 carbon atoms, or represents a group $-OR^8$, wherein

$R^8$ denotes hydrogen or straight-chain, branched or cyclic alkyl or alkenyl which has up to 16 carbon atoms and is optionally interrupted in the chain by an oxygen atom or a sulphur atom and/or is optionally substituted by one or more identical or different substituents from the group comprising halogen, cyano, hydroxyl, $C_2$-$C_7$-acyloxy and nitro, or by a group of the formula

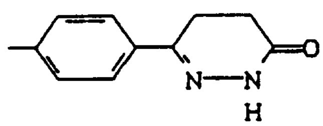

or by a phenyl, phenoxy, phenylthio or phenylsulphonyl group which is optionally substituted by halogen, cyano, di-$C_1$-$C_5$-alkylamino, $C_1$-$C_6$-alkoxy, $C_1$-$C_6$-alkyl, trifluoromethyl or nitro, or by a heteroaryl group of the series comprising pyridyl, thienyl, furyl, quinolyl and pyrimidyl, or by an amino group, this amino group carrying two identical or different substituents from the group comprising $C_1$-$C_6$-alxyl, $C_6$-$C_{14}$-aryl and $C_7$-$C_{14}$-aralKyl, or it being possible for these substituents, together with the nitrogen atom, optionally to form a 5- to 7-membered ring, which can contain, as a further hetero atom, an oxygen or sulphur atom or the N-phenyl or N-alkyl grouping, it being possible for the alkyl group to contain up to 4 carbon atoms,

and

$R^6$ represents a group of the formula

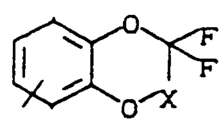

wherein

X denotes a direct bond or $-CF_2-$ or $-CHF-$, and physiologically acceptable salts thereof.

**2.** Compounds of the general formula (I) according to Claim 1, in which

$R^1$ represents hydrogen, nitro or a radical of the formula $-CO_2R^7$, wherein

$R^7$ denotes hydrogen or straight-chain or branched alkyl which has up to 12 carbon atoms and is optionally interrupted in the chain by an oxygen atom and/or is optionally mono- or polysubstituted by fluorine, chlorine, bromine, cyano, hydroxyl or acetoxy, or by a phenyl or phenoxy group which is optionally substituted by fluorine, chlorine, bromine, iodine, cyano, nitro, $C_1$-$C_4$-alkyl, $C_1$-$C_4$-alkoxy or trifluoromethyl, or by an $\alpha$-, $\beta$- or $\gamma$-pyridyl group, or by an amino group, this amino group carrying two identical or different substituents from the series comprising $C_1$-$C_4$-alkyl, phenyl and

benzyl,
or

$R^7$ represents a direct bond to $R^2$ (where $R^2 \neq$ phenyl),

$R^2$ and $R^4$ are identical or different and in each case represent straight-chain or branched alkyl with up to 6 carbon atoms, or represent phenyl or benzyl, or one of the substituents $R^2$ or $R^4$ represents straight-chain or branched alkyl which has up to 4 carbon atoms and is optionally mono- or polysubstituted by fluorine, chlorine, bromine, acetoxy, benzoyloxy, methoxy, hydroxyl, amino, phthalimido, aminoalkoxy or phthalimidoalkoxy with in each case up to 4 carbon atoms per alkoxy group,

$R^5$ represents straight-chain or branched alkyl with up to 4 carbon atoms, or represents the group $-OR^8$,
wherein

$R^8$ denotes hydrogen or straight-chain or branched alkyl which has up to 12 carbon atoms and is optionally interrupted in the chain by an oxygen atom and/or is optionally substituted by one or more substituents from the group comprising fluorine, chlorine, bromine, cyano, hydroxyl and acetoxy, or by the group of the formula

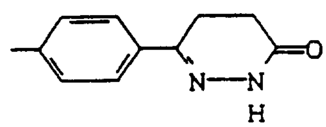

or by a phenyl or phenoxy group which is optionally substituted by fluorine, chlorine, bromine, iodine, cyano, nitro, $C_1$-$C_4$-alkyl, $C_1$-$C_4$-alkoxy or trifluoromethyl, or by an $\alpha$-, $\beta$- or $\gamma$-pyridyl group, or by an amino group, this amino group carrying two identical or different substituents from the series comprising $C_1$-$C_4$-alkyl, phenyl and benzyl,

and

$R^6$ represents a group of the formula

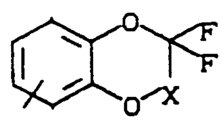

wherein

X denotes a direct bond or $-CF_2-$ or $-CHF-$,

and physiologically acceptable salts thereof.

**3.** Compounds of the general formula (I) according to Claim 1,
in which

$R^1$ represents nitro or a group of the formula $-CO_2R^7$,
wherein

$R^7$ denotes hydrogen or straight-chain or branched alkyl which has up to 8 carbon atoms and is optionally interrupted in the chain by an oxygen atom and/or is optionally substituted by up to 15 fluorine atoms, by chlorine, cyano, acetoxy, phenyl, phenoxy or $\alpha$-, $\beta$- or $\gamma$-pyridyl, or by an amino group, this amino group carrying two identical or different substituents from the group comprising $C_1$-$C_4$-alkyl and benzyl,
or

$R^7$ represents a direct bond to $R^2$ (where $R^2 \neq$ phenyl),

$R^2$ and $R^4$ are identical or different and in each case represent straight-chain or branched alkyl with up to 4 carbon atoms, or one of the substituents $R^2$ or $R^4$ represents alkyl which has up to 2 carbon atoms and is optionally substituted by up to 3 fluorine atoms or by chlorine, bromine, acetoxy, benzoyloxy, hydroxyl or aminoethoxy,

$R^5$ represents the group $-OR^8$,
wherein

$R^8$ denotes hydrogen or straight-chain or branched alkyl which has up to 8 carbon atoms and is optionally interrupted in the chain by an oxygen atom and/or is optionally substituted by up to 15 fluorine atoms, or by chlorine, cyano or acetoxy, or by the group of the formula

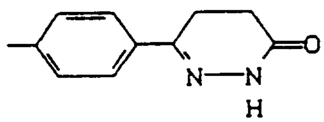

or by phenyl, phenoxy or α-, β- or γ-pyridyl, or by an amino group, this amino group carrying two identical or different substituents from the series comprising $C_1$-$C_4$-alkyl and benzyl,
and
$R^6$ represents a group of the formula

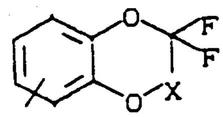

wherein
X represents a direct bond, or represents the groups $-CF_2-$ or $-CHF-$,
and physiologically acceptable salts thereof.

**4.** Process for the preparation of compounds of the general formula (I)

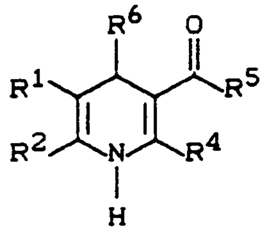

(I)

in which
$R^1$ represents hydrogen, cyano or nitro, or represents a radical of the formula $-CO_2R^7$, wherein
$R^7$ denotes hydrogen or straight-chain, branched or cyclic alkyl or alkenyl which has up to 16 carbon atoms and is optionally interrupted in the chain by an oxygen atom or a sulphur atom and/or is optionally substituted by one or more identical or different substituents from the group comprising halogen, cyano, hydroxyl, $C_2$-$C_7$-acyloxy and nitro or by a phenyl, phenoxy, phenylthio or phenylsulphonyl group which is optionally substituted by halogen, cyano, di-$C_1$-$C_5$-alkylamino, $C_1$-$C_6$-alkyl, $C_1$-$C_6$-alkoxy, trifluoromethyl or nitro, or by a heteroaryl group of the series comprising pyridyl, thienyl, furyl, quinolyl or pyrimidyl, or by an amino group, this amino group carrying two identical or different substituents from the group comprising $C_1$-$C_6$-alkyl, $C_6$-$C_{12}$-aryl and $C_7$-$C_{14}$-aralkyl, or it being possible for these substituents, together with the nitrogen atom, optionally to form a 5- to 7- membered ring which can contain, as a further hetero atom, a sulphur or oxygen atom or the N-phenyl or N-alkyl grouping, it being possible for the alkyl group to contain up to 4 carbon atoms,
or wherein
$R^7$ represents a direct bond to $R^2$ (where $R^2 \neq$ cyano, phenyl or formyl),
$R^2$ and $R^4$ are identical or different and in each case represent straight-chain, branched or cyclic

alkyl with up to 8 carbon atoms, or represent phenyl or benzyl, or one of the substituents $R^2$ or $R^4$ represents straight-chain or branched alkyl which has up to 6 carbon atoms and is substituted by halogen, $C_2$-$C_7$-acyloxy, hydroxyl, amino, phthalimido, alkoxy, dialkoxy, aminoalkoxy, phthalimidoalkoxy, piperidinoalkyl, morpholinoalkoxy or N-phenyl-N-piperazinoalkoxy with in each case up to 6 carbon atoms per alkoxy group, or represents formyl or cyano,

$R^5$ represents straight-chain, branched or cyclic alkyl with up to 8 carbon atoms, or represents a group $-OR^8$,

wherein

$R^8$ denotes hydrogen or straight-chain, branched or cyclic alkyl or alkenyl which has up to 16 carbon atoms and is optionally interrupted in the chain by an oxygen atom or a sulphur atom and/or is optionally substituted by one or more identical or different substituents from the group comprising halogen, cyano, hydroxyl, $C_2$-$C_7$-acyloxy and nitro, or by a group of the formula

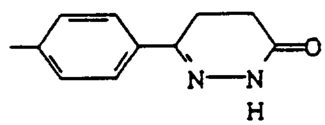

or by a phenyl, phenoxy, phenylthio or phenyl-sulphonyl group which is optionally substituted by halogen, cyano, di-$C_1$-$C_5$-alkylamino, $C_1$-$C_6$-alkoxy, $C_1$-$C_6$-alkyl, trifluoromethyl or nitro, or by a heteroaryl group of the series comprising pyridyl, thienyl, furyl, quinolyl and pyrimidyl, or by an amino group, this amino group carrying two identical or different substituents from the group comprising $C_1$-$C_6$-alkyl, $C_6$-$C_{14}$-aryl and $C_7$-$C_{14}$-aralkyl, or it being possible for these substituents, together with the nitrogen atom, optionally to form a 5- to 7-membered ring, which can contain, as a further hetero atom, an oxygen or sulphur atom or the N-phenyl or N-alkyl grouping, it being possible for the alkyl group to contain up to 4 carbon atoms,

and

$R^6$ represents a group of the formula

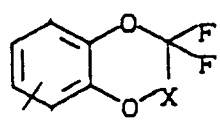

wherein

X denotes a direct bond or $-CF_2-$ or $-CHF-$,

characterised in that

[A] aldehydes of the general formula (II)

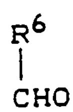

(II)

in which

$R^6$ has the meaning given,

and ketones of the general formula (III)

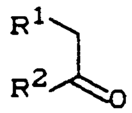

(III)

in which
$R^1$ and $R^2$ have the meaning given,
or Knoevenagel condensation products thereof (ylidene compounds) of the general formula (IV)

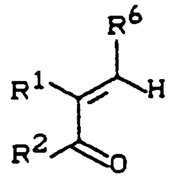

(IV)

in which
$R^1$, $R^2$ and $R^6$ have the abovementioned meaning, are reacted with enamines of the general formula (V)

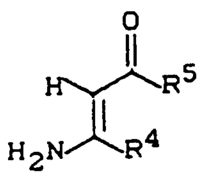

(V)

in which $R^4$ and $R^5$ have the meaning given,
in inert solvents, or in that
[B] aldehydes of the general formula (II) and ketones of the general formula (VI)

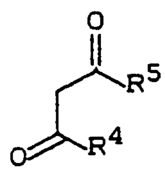

(VI)

in which
$R^4$ and $R^5$ have the meaning given,
or Knoevenagel condensation products thereof (ylidene compounds) of the general formula (VII)

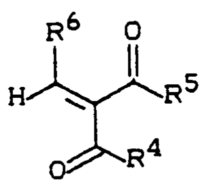

(VII)

in which
$R^4$, $R^5$ and $R^6$ have the abovementioned meaning,
are reacted with enamines of the general formula (VIII)

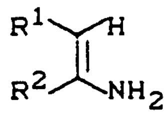

(VIII)

in which
$R^1$ and $R^2$ have the abovementioned meaning,
in inert solvents at temperatures between 20° and 150°C,
and in the case where $R^2$ represents a direct bond to $R^7$, the resulting dihydropyridines are cyclised to lactones.

**5.** Process for the preparation of dihydropyridine lactones of the formula (I), in which $R^2$ denotes a direct bond to $R^7$, characterised in that dihydropyridines of the general formula (I) in which

$R^1$ represents $CO_2R^7$,
$R^2$ represents an acyloxymethyl radical,
are cyclised in inert organic solvents, if appropriate in the presence of bases,
or
in which
$R^2$ represents a halogenomethyl radial,
are pyrolysed, if appropriate in the presence of solvents, at temperatures between 20 and 300°C,
the other substituents having the meaning given in Claim 4.

**6.** Compounds of the general formula (I) according to Claim 1, for use in combating circulatory diseases.

**7.** Medicaments containing at least one compound of the general formula (I) according to Claim 1.

**8.** Process for the preparation of medicaments, characterised in that compounds of the general formula (I) according to Claim 1 are converted into a suitable administration form, if appropriate using customary auxiliaries and excipients.

**9.** Use of compounds of the general formula (I) according to Claim 1 in the preparation of medicaments against circulatory diseases.

**10.** Aldehydes of the general formula (II)

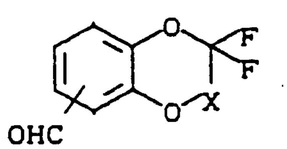

(II)

in which
X represents a direct bond, or represents a group of the formula $-CF_2-$ or $-CHF-$,
with the exception of the compounds 2,2-difluoro-5-formyl-1,3-benzodioxole and 3,4-trifluoroethylene-dioxybenzaldehyde.

**11.** 2,2-Difluoro-4-formyl-1,3-benzodioxole of the formula

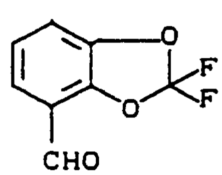

**12.** 2,2,3,3-Tetrafluoro-5-formyl-1,4-benzo-dioxane of the formula

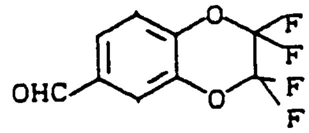

**13.** Process for the preparation of aldehydes of the general formula (II)

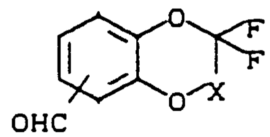

(II)

in which

X represents a direct bond, or
represents a group of the formula $-CF_2-$ or -CHF-,

with the exception of the compounds 2,2-difluoro-5-formyl-1,3-benzodioxole and 3,4-trifluoroethylendioxy-benzaldehyde,

characterised in that

[a] methyl-substituted compounds of the general formula (IX)

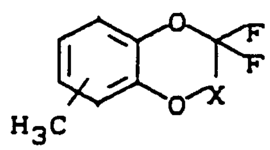

(IX)

in which

X has the abovementioned meaning,

are reacted in inert solvents with halogenating agents, optionally in the presence of free radical initiators,

and then the halogenomethyl compounds of the general formula (X)

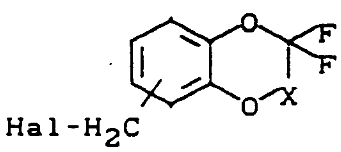

(X)

in which

X has the abovementioned meaning

and

Hal represents halogen,

are treated with urotropine, in inert solvents, optionally in the presence of acids,

or in that

[b] amino compounds of the general formula (XI)

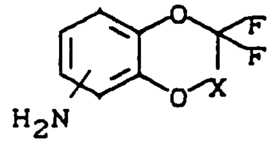

(XI)

in which
X has the abovementioned meaning,
are reacted with nitrites in inert solvents in the presence of acids,
and then the diazonium salts obtained are reacted with formaldoxime.

**Revendications**

**1.** Dioxyalkylènearyl-dihydropyridines de formule générale (I)

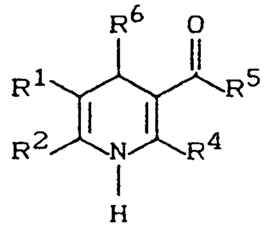

(I)

dans laquelle

$R^1$

- représente l'hydrogène, un groupe cyano, un groupe nitro ou
- un reste de formule $-CO_2R^7$,
  où

$R^7$

- est l'hydrogène ou un groupe alkyle ou alcényle à chaîne droite, à chaîne ramifiée ou cyclique ayant jusqu'à 16 atomes de carbone, dont la chaîne est interrompue le cas échéant par un atome d'oxygène ou un atome de soufre et/ou qui est substitué le cas échéant par un ou plusieurs radicaux, identiques ou différents, du groupe halogéno, cyano, hydroxy, acyloxy en $C_2$ à $C_7$, nitro ou par un groupe phényle, phénoxy, phénylthio ou phénylsulfonyle éventuellement substitué par un halogène, un radical cyano, di(alkyle en $C_1$ à $C_5$)amino, alkyle en $C_1$ à $C_6$, alkoxy en $C_1$ à $C_6$, trifluorométhyle ou nitro ou par un groupe hétéroaryle de la série pyridyle, thiényle, furyle, quinolyle ou pyrimidyle, ou par un groupe amino, ce groupe amino portant éventuellement des substituants identiques ou différents du groupe alkyle en $C_1$ à $C_6$, aryle en $C_6$ à $C_{12}$, aralkyle en $C_7$ à $C_{14}$, ou bien ces substituants forment éventuellement avec l'atome d'azote un noyau pentagonal à heptagonal qui peut comporter comme autre hétéro-atome un atome de soufre ou d'oxygène ou le groupement N-phényle ou N-alkyle, le groupe alkyle pouvant comprendre jusqu'à 4 atomes de carbone

ou dans laquelle

$R^7$

- est une liaison directe à $R^2$ (pour $R^2 \neq$ cyano, phényle ou formyle),

$R^2$, $R^4$

- sont identiques ou différents et représentent chacun
- un groupe alkyle à chaîne droite, à chaîne ramifiée ou cyclique ayant jusqu'à 8 atomes de carbone,
- un groupe phényle ou benzyle, ou bien
- l'un des substituants $R^2$ ou $R^4$ représente un groupe alkyle à chaîne droite ou ramifiée ayant jusqu'à 6 atomes de carbone, qui est substitué par un halogène, un radical acyloxy en $C_2$ à $C_7$, hydroxy, amino, phtalimido, alkoxy, dialkoxy, aminoalkoxy, phtalimidoalkoxy, pipéridinoalkyle, morpholinoalkoxy ou N-phényl-N-pipérazinoalkoxy avec dans chaque cas jusqu'à 6 atomes de carbone par groupe alkoxy, ou bien un groupe formyle ou cyano,

$R^5$
- est un groupe alkyle à chaîne droite, à chaîne ramifiée ou cyclique ayant jusqu'à 8 atomes de carbone, ou bien
- un groupe $-OR^8$, dans lequel

$R^8$
- représente l'hydrogène ou un groupe alkyle ou alcényle à chaîne droite, à chaîne ramifiée ou cyclique ayant jusqu'à 16 atomes de carbone, dont la chaîne est éventuellement interrompue par un atome d'oxygène ou un atome de soufre et/ou qui est éventuellement substitué par un ou plusieurs radicaux identiques ou différents du groupe des radicaux halogéno, cyano, hydroxy, acyloxy en $C_2$ à $C_7$, nitro ou par un groupe de formule

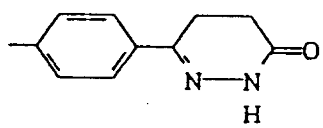

ou par un groupe phényle, phénoxy, phénylthio ou phénylsulfonyle portant éventuellement un substituant halogéno, cyano, di(alkyle en $C_1$ à $C_5$)-amino, alkoxy en $C_1$ à $C_6$, alkyle en $C_1$ à $C_6$, trifluorométhyle ou nitro ou par un groupe hétéroaryle de la série pyridyle, thiényle, furyle, quinolyle ou pyrimidyle ou par un groupe amino, ce groupe amino pouvant porter deux substituants identiques ou différents du groupe alkyle en $C_1$ à $C_6$, aryle en $C_6$ à $C_{14}$, aralkyle en $C_7$ à $C_{14}$, ou bien ces substituants forment le cas échéant avec l'atome d'azote un noyau pentagonal à heptagonal qui peut comporter comme autre hétéro-atome un atome d'oxygène ou de soufre ou le groupement N-phényle ou N-alkyle, le groupement alkyle pouvant comprendre jusqu'à 4 atomes de carbone,

et

$R^6$
- est un groupe de formule

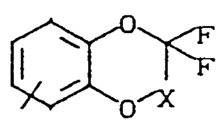

dans laquelle

X
- est une liaison directe ou un groupe $-CF_2-$ ou $-CHF-$,

ainsi que leurs sels acceptables du point de vue physiologique.

**2.** Composés de formule générale (I) suivant la revendication 1,
dans laquelle

$R^1$
- est l'hydrogène, un groupe nitro ou un reste de formule $-CO_2R^7$, dans laquelle

$R^7$
- représente l'hydrogène, un groupe alkyle à chaîne droite ou ramifiée ayant jusqu'à 12 atomes de carbone, dont la chaîne est éventuellement interrompue par un atome d'oxygène et/ou qui est substitué le cas échéant une ou plusieurs fois par du fluor, du chlore, du brome, un groupe cyano, hydroxy, acétyloxy, ou par un groupe phényle ou phénoxy éventuellement substitué par du fluor, du chlore, du brome, de l'iode, un radical cyano, nitro, alkyle en $C_1$ à $C_4$, alkoxy en $C_1$ à $C_4$ ou trifluoromé-

thyle, ou par un groupe α-, β-, ou γ-pyridyle, ou par un groupe amino, ce groupe amino portant deux substituants identiques ou différents de la série alkyle en $C_1$ à $C_4$, phényle ou benzyle,

ou bien

$R^7$

- représente une liaison directe au groupe $R^2$ (pour $R^2 \neq$ phényle),

$R^2$, $R^4$

- sont identiques ou différents et représentent chacun
- un groupe alkyle à chaîne droite ou ramifiée ayant jusqu'à 6 atomes de carbone, un groupe phényle ou un groupe benzyle ou bien
- l'un des substituants $R^2$ ou $R^4$ représente un groupe alkyle à chaîne droite ou ramifiée ayant jusqu'à 4 atomes de carbone, qui est substitué le cas échéant une ou plusieurs fois par du fluor, du chlore, du brome, un radical acétyloxy, benzoyloxy, méthoxy, hydroxy, amino, phtalimido, aminoalkoxy ou phtalimidoalkoxy avec dans chaque cas jusqu'à 4 atomes de carbone par groupe alkoxy,

$R^5$

- est un groupe alkyle à chaîne droite ou ramifiée ayant jusqu'à 4 atomes de carbone, ou bien
- le groupe $-OR^8$,
  dans lequel

$R^8$

- représente l'hydrogène ou un groupe alkyle à chaîne droite ou ramifiée ayant jusqu'à 12 atomes de carbone, dont la chaîne est éventuellement interrompue par un atome d'oxygène et/ou qui est éventuellement substitué par un ou plusieurs substituants du groupe fluor, chlore, brome, cyano, hydroxy, acétyloxy ou par le groupe de formule

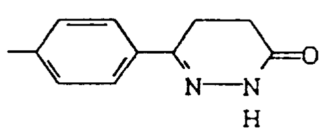

,

ou par un groupe phényle ou phénoxy éventuellement substitué par du fluor, du chlore, du brome, de l'iode, un radical cyano, nitro, alkyle en $C_1$ à $C_4$, alkoxy en $C_1$ à $C_4$ ou trifluorométhyle, ou par un groupe α-, β-, ou γ-pyridyle, ou par un groupe amino, ce groupe amino portant deux substituants identiques ou différents de la série alkyle en $C_1$ à $C_4$, phényle ou benzyle

et

$R^6$

- représente un groupe de formule

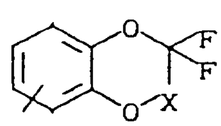

dans laquelle

X

- est une liaison directe ou un groupe $-CF_2-$ ou $-CHF-$,

ainsi que leurs sels acceptables du point de vue physiologique.

**3.** Composés de formule générale (I) suivant la revendication 1,
dans laquelle

$R^1$

- est un groupe nitro ou un groupe de formule $-CO_2R^7$,
  dans laquelle

$R^7$

- est l'hydrogène ou un groupe alkyle à chaîne droite ou ramifiée ayant jusqu'à 8 atomes de carbone, dont la chaîne est éventuellement interrompue par un atome d'oxygène et/ou qui est éventuellement substitué par jusqu'à 15 atomes de fluor, par du chlore, un radical cyano, acétyloxy, phényle, phénoxy, $\alpha$-, $\beta$- ou $\gamma$-pyridyle ou par un groupe amino, ce groupe amino portant deux substituants identiques ou différents de la série alkyle en $C_1$ à $C_4$ ou benzyle,

ou bien

$R^7$

- représente une liaison directe au groupe $R^2$ (pour $R^2 \neq$ phényle),

$R^2$, $R^4$

- sont identiques ou différents et représentent chacun
- un groupe alkyle à chaîne droite ou ramifiée ayant jusqu'à 4 atomes de carbone, ou bien
- l'un des substituants $R^2$ ou $R^4$ représente un groupe alkyle ayant jusqu'à 2 atomes de carbone, qui est éventuellement substitué par jusqu'à 3 atomes de fluor, par du chlore, du brome, un groupe acétoxy, benzoyloxy, hydroxy, ou aminoéthoxy,

$R^5$

- représente le groupe $-OR^8$, dans lequel

$R^8$

- est l'hydrogène ou un groupe alkyle à chaîne droite ou ramifiée ayant jusqu'à 8 atomes de carbone, dont la chaîne est éventuellement interrompue par un atome d'oxygène et/ou qui est éventuellement substitué par jusqu'à 15 atomes de fluor, par du chlore, un radical cyano, acétyloxy ou par le groupe de formule

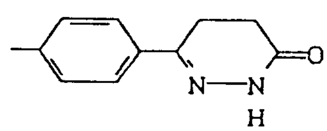

ou par un groupe phényle, phénoxy, $\alpha$-, $\beta$- ou $\gamma$-pyridyle ou par un groupe amino, ce groupe amino portant deux substituants identiques ou différents de la série alkyle en $C_1$ à $C_4$ ou benzyle,

et

$R^6$

- représente un groupe de formule

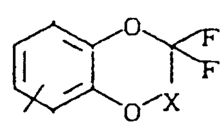

dans laquelle

X

- représente une liaison directe, ou bien
- les groupes $-CF_2-$ et $-CHF-$,

ainsi que leurs sels acceptables du point de vue physiologique.

**4.** Procédé de production de composés de formule générale (I)

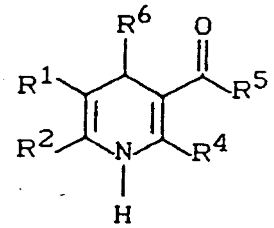

(I)

dans laquelle

$R^1$
- est l'hydrogène, un groupe cyano, nitro ou représente
- un reste de formule $-CO_2R^7$, dans laquelle

$R^7$
- est l'hydrogène ou un groupe alkyle ou alcényle à chaîne droite, à chaîne ramifiée ou cyclique ayant jusqu'à 16 atomes de carbone, dont la chaîne est éventuellement interrompue par un atome d'oxygène ou par un atome de soufre et/ou qui est substitué le cas échéant par un ou plusieurs substituants identiques ou différents du groupe halogéno, cyano, hydroxy, acyloxy en $C_2$ à $C_7$, nitro ou par un groupe phényle, phénoxy, phénylthio ou phénylsulfonyle éventuellement substitué par un halogène, un radical cyano, di(alkyle en $C_1$ à $C_5$)amino, alkyle en $C_1$ à $C_6$, alkoxy en $C_1$ à $C_6$, trifluorométhyle ou nitro, ou par un groupe hétéroaryle de la série pyridyle, thiényle, furyle, quinolyle ou pyrimidyle, ou par un groupe amino, ce groupe amino portant deux substituants identiques ou différents du groupe alkyle en $C_1$ à $C_6$, aryle en $C_6$ à $C_{12}$, aralkyle en $C_7$ à $C_{14}$, ou bien ces substituants formant le cas échéant avec l'atome d'azote un noyau pentagonal à heptagonal qui peut comporter comme autre hétéro-atome un atome de soufre ou un atome d'oxygène ou le groupement N-phényle ou N-alkyle, le groupe alkyle pouvant comprendre jusqu'à 4 atomes de carbone

ou dans laquelle

$R^7$
- représente une liaison directe à $R^2$ (pour $R^2 \neq$ cyano, phényle ou formyle),

$R^2$, $R^4$
- sont identiques ou différents et représentent chacun
- un groupe alkyle à chaîne droite, à chaîne ramifiée ou cyclique ayant jusqu'à 8 atomes de carbone,
- un groupe phényle ou benzyle, ou bien
- l'un des substituants $R^2$ ou $R^4$ représente un groupe alkyle à chaîne droite ou à chaîne ramifiée ayant jusqu'à 6 atomes de carbone, qui est substitué par un halogène, un groupe acyloxy en $C_2$ à $C_7$, hydroxy, amino, phtalimido, alkoxy, dialkoxy, aminoalkoxy, phtalimidoalkoxy, pipéridinoalkyle, morpholinoalkoxy ou N-phényl-N-pipérazinoalkoxy ayant chacun jusqu'à 6 atomes de carbone par groupe alkoxy, ou représente un groupe formyle ou cyano,

$R^5$
- est un groupe alkyle à chaîne droite, à chaîne ramifiée ou cyclique ayant jusqu'à 8 atomes de carbone,
  ou bien
- un groupe de formule $-OR^8$,

dans laquelle

$R^8$
- représente l'hydrogène ou un groupe alkyle ou alcényle à chaîne droite, à chaîne ramifiée ou cyclique ayant jusqu'à 16 atomes de carbone, dont la chaîne est interrompue le cas échéant par un atome d'oxygène ou par un atome de soufre et/ou qui est éventuellement substitué par un ou plusieurs substituants identiques ou différents du groupe halogéno, cyano, hydroxy, acyloxy en $C_2$ à $C_7$, nitro, ou par un

groupe de formule

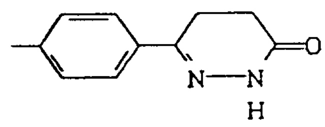

ou par un groupe phényle, phénoxy, phénylthio ou phénylsulfonyle éventuellement substitué par un halogène, un radical cyano, di(alkyle en $C_1$ à $C_5$)amino, alkoxy en $C_1$ à $C_6$, alkyle en $C_1$ à $C_6$, trifluorométhyle ou nitro, ou par un groupe hétéroaryle de la série pyridyle, thiényle, furyle, quinolyle ou pyrimidyle, ou par un groupe amino, ce groupe amino portant deux substituants identiques ou différents de la série alkyle en $C_1$ à $C_6$, aryle en $C_6$ à $C_{14}$, aralkyle en $C_7$ à $C_{14}$, ou bien ces substituants formant éventuellement avec l'atome d'azote un noyau pentagonal à heptagonal qui peut contenir comme autre hétéro-atome un atome d'oxygène ou un atome de soufre ou le groupement N-phényle ou N-alkyle, le groupe alkyle pouvant comprendre jusqu'à 4 atomes de carbone,

et

$R^6$

- est un groupe de formule

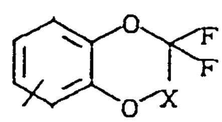

dans laquelle

X

- est une liaison directe ou un groupe $-CF_2-$ ou -CHF-,

caractérisé en ce qu'on fait réagir

[A] des aldéhydes de formule générale (II),

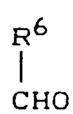

(II)

dans laquelle

$R^6$ a la définition indiquée,

et des cétones de formule générale (III)

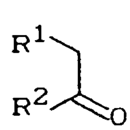

(III),

dans laquelle

$R^1$ et $R^2$ ont la définition indiquée,

ou leurs produits de condensation de Knoevenagel (composés du type ylidène) de formule générale (IV),

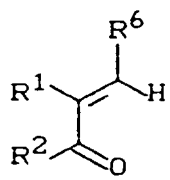

(IV)

dans laquelle

$R^1$, $R^2$, $R^6$ ont la définition indiquée ci-dessus, avec des énamines de formule générale (V),

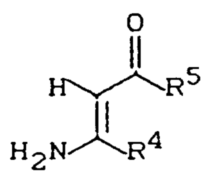

(V),

dans laquelle $R^4$ et $R^5$ ont la définition indiquée,

dans des solvants inertes,

ou bien

[B] des aldéhydes de formule générale (II) et des cétones de formule générale (VI),

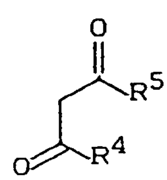

(VI),

où

$R^4$ et $R^5$ ont la définition indiquée,

ou leurs produits de condensation de Knoevenagel (composés du type ylidène) de formule générale (VII)

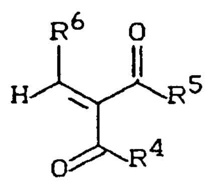

(VII),

dans laquelle

$R^4$, $R^5$, $R^6$ ont la définition indiquée ci-dessus avec des énamines de formule générale (VIII)

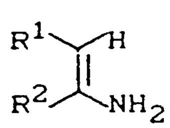

(VIII)

dans laquelle

$R^1$ et $R^2$ ont la définition indiquée ci-dessus, dans des solvants inertes, à des températures comprises entre 20 et 150 °C,

et au cas où $R^2$ représente une liaison directe à $R^7$, on cyclise en lactones les dihydropyridines

obtenues.

**5.** Procédé de production de dihydropyridine-lactones de formule (I), dans lesquelles $R^2$ représente une liaison directe à $R^7$, caractérisé en ce qu'on cyclise dans des solvants organiques inertes, éventuellement en présence de bases, des dihydropyridines de formule générale (I),
dans laquelle
$R^1$ représente un groupe $CO_2R^7$,
$R^2$ est un reste acyloxyméthyle,
ou bien
dans laquelle
$R^2$ représente un reste halogénométhyle,
et on les pyrolyse, le cas échéant en présence de solvants, à des températures comprises entre 20 et 300 °C,
les autres substituants ayant la définition indiquée dans la revendication 4.

**6.** Composés de formule générale (I) suivant la revendication 1, destinés à être utilisés pour combattre des maladies du système circulatoire.

**7.** Médicaments contenant au moins un composé de formule générale (I) suivant la revendication 1.

**8.** Procédé de préparation de médicaments, caractérisé en ce qu'on confère la forme d'application appropriée à des composés de formule générale (I) suivant la revendication 1, éventuellement en utilisant des substances auxiliaires et des supports classiques.

**9.** Utilisation de composés de formule générale (I) suivant la revendication 1 dans la préparation de médicaments contre des maladies du système circulatoire.

**10.** Aldéhydes de formule générale (II)

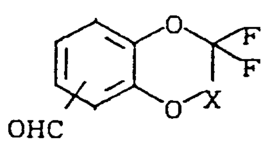

(II)

dans laquelle
X
- est une liaison directe, ou bien
- un groupe de formule $-CF_2-$ ou $-CHF-$,

à l'exception des composés 2,2-difluoro-5-formyl-1,3-benzodioxole et 3,4-trifluoréthylènedioxybenzaldéhyde.

**11.** Le 2,2-difluoro-4-formyl-1,3-benzodioxole de formule

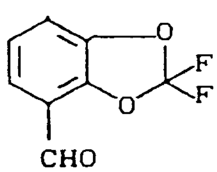

**12.** Le 2,2,3,3-tétrafluoro-5-formyl-1,4-benzodioxanne de formule

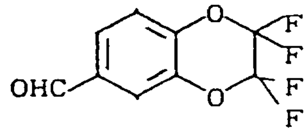

**13.** Procédé de production d'aldéhydes de formule générale (II)

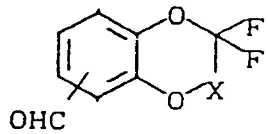

(II)

dans laquelle

X représente une liaison directe, ou bien un groupe de formule $-CF_2-$ ou $-CHF-$,

excepté les composés 2,2-difluoro-5-formyl-1,3-benzodioxole et 3,4-trifluoréthylènedioxy-benzaldéhyde, caractérisé en ce que

[a] On fait réagir des composés à substituant méthyle de formule générale (IX)

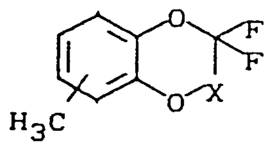

(IX)

dans laquelle

X a la définition indiquée ci-dessus, dans des solvants inertes, avec des agents d'halogénation, le cas échéant en présence de générateurs de radicaux, puis on traite à l'urotropine dans des solvants inertes, éventuellement en présence d'acides, les composés halogénométhyliques de formule générale (X)

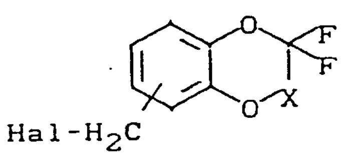

(X)

dans laquelle

X a la définition indiquée ci-dessus et

Hal représente un halogène,

ou bien

[b] On fait réagir avec des nitrites dans des solvants inertes, en présence d'acides, des composés aminés de formule générale (XI)

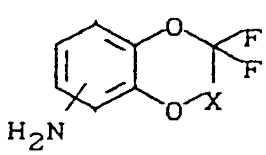

(XI)

dans laquelle

X a la définition indiquée ci-dessus, puis on fait réagir avec la formaldoxime les sels de diazonium obtenu.